(19) Europäisches Patentamt / European Patent Office / Office européen des brevets

(11) EP 2 582 242 B1

(12) EUROPÄISCHE PATENTSCHRIFT

(45) Veröffentlichungstag und Bekanntmachung des Hinweises auf die Patenterteilung:
**28.12.2016 Patentblatt 2016/52**

(21) Anmeldenummer: **11725462.3**

(22) Anmeldetag: **16.06.2011**

(51) Int Cl.:
***A01N 43/56*** *(2006.01)* ***A01N 43/713*** *(2006.01)*
***A61K 31/4439*** *(2006.01)* ***A01P 7/00*** *(2006.01)*

(86) Internationale Anmeldenummer:
**PCT/EP2011/059988**

(87) Internationale Veröffentlichungsnummer:
**WO 2011/157778 (22.12.2011 Gazette 2011/51)**

(54) **WIRKSTOFFKOMBINATIONEN MIT INSEKTIZIDEN UND AKARIZIDEN EIGENSCHAFTEN**

COMBINATIONS OF ACTIVE SUBSTANCES WITH INSECTICIDAL AND ACARICIDAL PROPERTIES

COMBINAISONS D'AGENTS A PROPRIETES INSECTICIDES ET ACARICIDES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.06.2010 US 356224 P**
**18.06.2010 EP 10166439**

(43) Veröffentlichungstag der Anmeldung:
**24.04.2013 Patentblatt 2013/17**

(73) Patentinhaber: **Bayer Intellectual Property GmbH**
**40789 Monheim (DE)**

(72) Erfinder:
- **FUNKE, Christian**
  **42799 Leichlingen (DE)**
- **HUNGENBERG, Heike**
  **40764 Langenfeld (DE)**
- **FISCHER, Rüdiger**
  **50259 Pulheim (DE)**

(74) Vertreter: **BIP Patents**
**c/o Bayer Intellectual Property GmbH**
**Alfred-Nobel-Straße 10**
**40789 Monheim am Rhein (DE)**

(56) Entgegenhaltungen:
**WO-A1-2005/048713 WO-A1-2005/053406**
**WO-A1-2007/144100 WO-A1-2008/072783**
**WO-A2-2004/046129 WO-A2-2005/053393**
**WO-A2-2007/017433 WO-A2-2011/098408**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue Wirkstoffkombinationen, die aus den Verbindungen der Formel (1-1) in Kombination mit weiteren insektiziden Wirkstoffen (II) bestehen und sehr gut zur Bekämpfung von tierischen Schädlingen wie Insekten und / oder unerwünschten Akariden geeignet sind.

Die Verbindungen der Formel (1-1) sind teils aus WO 2007/144100 bekannt und ihre insektizide Wirkung beschrieben. Die in dieser Beschreibung mit ihrem "common name" genannten Wirkstoffe sind beispielsweise aus "The Pesticide Manual" 14th Ed., British Crop Protection Council 2006, und der Webseite http://www.alanwood.net/pesticides bekannt.

Die akarizide und / oder insektizide Wirksamkeit und / oder Wirkungsbreite und / oder die Pflanzenverträglichkeit der bekannten Verbindungen, insbesondere gegenüber Kulturpflanzen, ist jedoch nicht immer ausreichend.

Es wurde nun gefunden, dass Wirkstoffkombinationen enthaltend die Verbindungen der allgemeinen Formel (1-1),

(I),

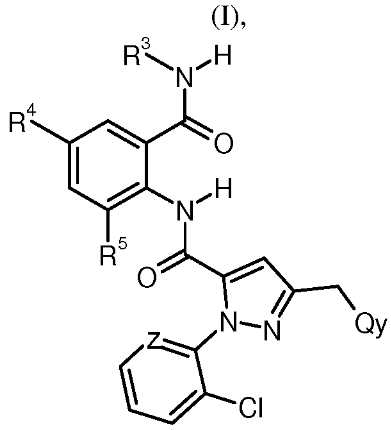

(I-1)

in welcher

$R^3$ für einen der folgenden Reste steht

H

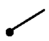 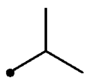 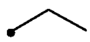 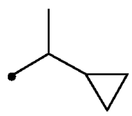

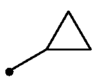 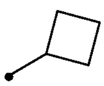 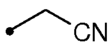 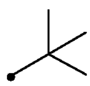 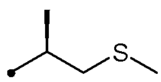

$R^4$ für Chlor und Cyano steht,

$R^4$ ebenfalls für Brom, Fluor, Jod oder Methyl steht,

$R^5$ für Methyl steht,

Z für N oder CH steht,

Qy für einen gegebenenfalls einfach oder mehrfach gleich oder verschieden substituierten heteroaromatischen Ring der Reihe Q-58 und Q-59 steht,

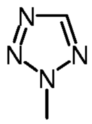
Q-58

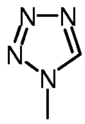
Q-59 ,

wobei die Substituenten unabhängig voneinander ausgewählt sein können aus Methyl, Ethyl, cyclo-Propyl, tert-Butyl, Difluormethyl, Trifluormethyl, Pentafluorethyl., n-Heptafluorpropyl und iso-Heptafluorpropyl,
wobei die Verbindungen der Formel (1-1) in Form von Salzen vorliegen können,
und wobei die Wirkstoffkombinationen eine Mischung von Verbindungen der allgemeinen Formel (1-1) enthalten, in welchen Qy für Q58 und Q59 steht,
und ein oder mehrere weitere Insektizide und / oder Akarizide ausgewählt aus der Gruppe (II) bestehend aus

| |
|---|
| Acrinathrin |
| Alpha-Cypermethrin |
| Betacyfluthrin |
| Cyhalothrin |
| Cypermethrin |
| Deltamethrin |
| Esfenvalerat |
| Etofenprox |
| Fenpropathrin |
| Fenvalerat |
| Flucythrinat |
| Lambda-Cyhalothrin |
| Gamma-Cyhalothrin |
| Permethrin |
| Tau-fluvalinat |
| Transfluthrin |
| Zeta-Cypermethrin |
| Cyfluthrin |
| Bifenthrin |
| Tefluthrin |
| Eflusilanat |
| Fubfenprox |
| Pyrethrin |
| Resmethrin |
| Imidacloprid |
| Acetamiprid |
| Thiamethoxam |
| Nitenpyram |
| Thiacloprid |
| Dinotefuran |

(fortgesetzt)

| |
|---|
| Clothianidin |
| Imidaclothiz |
| Chlorfluazuron |
| Diflubenzuron |
| Lufenuron |
| Teflubenzuron |
| Triflumuron |
| Novaluron |
| Flufenoxuron |
| Hexaflumuron |
| Bistrifluoron |
| Noviflumuron |
| Buprofezin |
| Cyromazine |
| Methoxyfenozide |
| Tebufenozide |
| Halofenozide |
| Chromafenozide |
| Endosulfan |
| Fipronil |
| Ethiprole |
| Pyrafluprole |
| Pyriprole |
| Flubendiamide |
| Chlorantraniliprol e (Rynaxypyr) |
| Cyazypyr |
| Emamectin |
| Emamectin |
| benzoate |
| Abamectin |
| Ivermectin |
| Milbemectin |
| Lepimectin |
| Tebufenpyrad |
| Fenpyroximat |
| Pyridaben |
| Fenazaquin |
| Pyrimidifen |
| Tolfenpyrad |

(fortgesetzt)

| |
|---|
| Dicofol |
| Cyenopyrafen |
| Cyflumetofen |
| Acequinocyl |
| Fluacrypyrin |
| Bifenazate |
| Diafenthiuron |
| Etoxazole |
| Clofentezine |
| Spinosad |
| Triarathen |
| Tetradifon |
| Propargit |
| Hexythiazox |
| Bromopropylat |
| Chinomethionat |
| Amitraz |
| Pyrifluquinazone |
| Pymetrozine |
| Flonicamid |
| Pyriproxyfen |
| Diofenolan |
| Chlorfenapyr |
| Metaflumizone |
| Indoxacarb |
| Chlorpyrifos |
| Spirodiclofen |
| Spiromesifen |
| Spirotetramat |
| Pyridalyl |
| Spinetoram |
| Acephate |
| Triazophos |
| Profenofos |
| Fenamiphos |
| 4-{[(6-Chlorpyrid-3-yl)methyl] (2,2-difluorethyl) amin o}furan-2(5H)-on |
| Cadusaphos |
| Carbaryl |
| Carbofuran |

(fortgesetzt)

| |
|---|
| Ethoprophos |
| Thiodicarb |
| Aldicarb |
| Metamidophos |
| Methiocarb |
| Sulfoxaflor |
| Bacillus firmus I-1582 |
| Dichloropropen |
| Dimethoate |
| Metaldehyd |
| Methomyl |
| Cartap |
| Öl (beispielsweise Petroleum) |
| Chloropicrin |
| Carbosulfan |
| Dichlorvos |
| Metam-Natrium |
| Phoxim |
| Monocrotophos |
| Oxamyl |
| Methidathion |
| Fenitrothion |
| Terbufos |
| Fluensulfone |
| Imicyafos |
| 11-(4-Chlor-2,6-dimethylphenyl)-12-hydroxy-1,4-dioxa-9-azadispiro[4.2.4.2 ] tetradec-11-en-10-on |
| 2-{6-[2-(5-Fluorpyridin-3-yl)-1,3-thiazol-5-yl]pyridin-2-yl}pyrimidin |

Wenn im Rahmen dieser Beschreibung die Kurzform des "common name" eines Wirkstoffes verwendet wird, so sind damit jeweils alle gängigen Derivate, wie die Ester und Salze, und Isomere, insbesondere optische Isomere umfasst, insbesondere die handelsübliche Form bzw. Formen. Wird mit dem "common name" ein Ester oder Salz bezeichnet, so sind damit auch jeweils alle anderen gängigen Derivate wie andere Ester und Salze, die freien Säuren und Neutralverbindungen, und Isomere, insbesondere optische Isomere umfasst, insbesondere die handelsübliche Form bzw. Formen. Die angegebenen chemischen Verbindungsnamen bezeichnen zumindest ein der von dem "common name" umfassten Verbindungen, häufig eine bevorzugte Verbindung.

Überraschenderweise ist die insektizide und / oder akarizide Wirkung der erfindungsgemäßen Wirkstoffkombinationen wesentlich höher als die Summe der Wirkungen der einzelnen Wirkstoffe. Es liegt ein nicht vorhersehbarer echter synergistischer Effekt vor und nicht nur eine Wirkungsergänzung.

Die Verbindungen der Formel (I-1) liegen in Form verschiedener Regioisomere vor, d.h. in Form von Mischungen aus Verbindungen mit der Definition aus Q58 und Q59. Erfindungsgemäß umfasst sind daher Wirkstoffkombinationen enthaltend Mischungen aus Verbindungen der Formel (1-1), wobei $Q_Y$ die Bedeutungen Q58 und Q59 hat und die Verbindungen in verschiedenen Mischungsverhältnissen vorliegen können, und einen oder mehrere Wirkstoffe aus der Gruppe (II). Bevorzugt sind dabei Mischungsverhältnisse aus Verbindungen der Formel (1-1), worin der Rest $Q_Y$ für Q58 steht zu Verbindungen der Formel (1-1) worin der Rest Qy für Q59 steht, von 60:40 bis 99:1, besonders bevorzugt

von 70:30 bis 97:3, ganz besonders bevorzugt von 80:20 bis 95:5. Insbesonders bevorzugt sind die folgenden Mischungsverhältnisse einer Verbindung der Formel (1-1), wobei $Q_Y$ die Bedeutung Q58 hat zur Verbindung der Formel (I), wobei $Q_Y$ die Bedeutung Q59 hat: 80:20; 81:19; 82:18; 83:17; 84:16; 85:15, 86:14; 87:13; 88:12; 89:11; 90:10, 91:9; 92:8; 93:7; 96:6; 95;5.

Weiterhin ebenfalls bevorzugt sind Wirkstoffkombinationen enthaltend Wirkstoffe der allgemeinen Formel (1-1) in der Qy für Q58 und Q59 steht und einen Wirkstoff der Gruppe (II) ausgewählt aus

| |
|---|
| Bacillus firmus 1-1582 |
| Dichloropropen |
| Dimethoate |
| Metaldehyd |
| Methomyl |
| Cartap |
| Öl (beispielsweise Petroleum) |
| Chloropicrin |
| Carbosulfan |
| Dichlorvos |
| Metam-Natrium |
| Phoxim |
| Monocrotophos |
| Oxamyl |
| Methidathion |
| Fenitrothion |
| Terbufos |
| Fluensulfone |
| Imicyafos |
| 11-(4-Chlor-2,6-dimethylphenyl)-12-hydroxy-1,4-dioxa-9-azadispiro[4.2.4.2]tetra dec-11-en-10-on |
| 2-{6-[2-(5-Fluorpyridin-3-yl)-1,3-thiazol-5-yl]pyridin-2-yl}pyrimidin |

Weiterhin besonders bevorzugt sind Wirkstoffkombinationen enthaltend Wirkstoffe der allgemeinen Formel (I-1) in der Qy für Q58 und Q59 steht und einen Wirkstoff der Gruppe (II) ausgewählt aus

| |
|---|
| Acrinathrin |
| Alpha-Cypermethrin |
| Betacyfluthrin |
| Cyhalothrin |
| Cypermethrin |
| Deltamethrin |
| Lambda-Cyhalothrin |
| Gamma-Cyhalothrin |
| Transfluthrin |
| Cyfluthrin |
| Bifenthrin |

(fortgesetzt)

| |
|---|
| Tefluthrin |
| Imidacloprid |
| Acetamiprid |
| Thiamethoxam |
| Thiacloprid |
| Dinotefuran |
| Clothianidin |
| Lufenuron |
| Triflumuron |
| Novaluron |
| Flufenoxuron |
| Buprofezin |
| Methoxyfenozide |
| Tebufenozide |
| Fipronil |
| Ethiprole |
| Flubendiamide |
| Chlorantraniliprole (Rynaxypyr) |
| Cyazypyr |
| Emamectin |
| Emamectin benzoate |
| Abamectin |
| Milbemectin |
| Tebufenpyrad |
| Fenpyroximat |
| Diafenthiuron |
| Spinosad |
| Flonicamid |
| Chlorfenapyr |
| Metaflumizone |
| Indoxacarb |
| Chlorpyrifos |
| Spirodiclofen |
| Spiromesifen |
| Spirotetramat |
| Pyridalyl |
| Spinetoram |
| Acephate |
| Triazophos |

(fortgesetzt)

| |
|---|
| Profenofos |
| Fenamiphos |
| 4-{[(6-Chlorpyrid-3-yl)methyl](2,2-difluorethyl)amino}furan-2(5H)-on |
| Cadusaphos |
| Carbaryl |
| Carbofuran |
| Ethoprophos |
| Thiodicarb |
| Aldicarb |
| Metamidophos |
| Methiocarb |
| Sulfoxaflor |

Weiterhin ebenfalls besonders bevorzugt sind Wirkstoffkombinationen enthaltend Wirkstoffe der allgemeinen Formel (I-1) in der Qy für Q58 und Q59 steht und einen Wirkstoff der Gruppe (II) ausgewählt aus

| |
|---|
| Bacillus firmus 1-1582 |
| Dichloropropen |
| Dimethoate |
| Methomyl |
| Imicyafos |
| Fluensulfone |
| 11-(4-Chlor-2,6-dimethylphenyl)-12-hydroxy-1,4-dioxa-9-azadispiro[4.2.4.2]tetradec-11-en-10-on |
| 2-{6-[2-(5-Fluorpyridin-3-yl)-1,3-thiazol-5-yl]pyridin-2-yl}pyrimidin |

Ganz besonders bevorzugt sind Wirkstoffkombinationen enthaltend wirkstoffe der Formel (I-1-1) bis (I-1-60) in der Qy für Q58 und Q59 steht und einen oder mehrere Wirkstoffe der Gruppe (II).

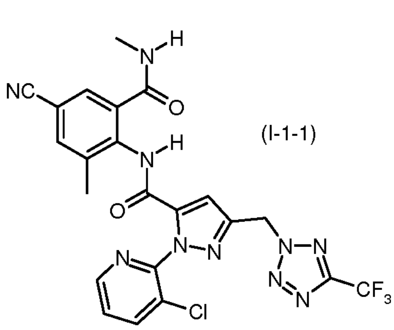
(I-1-1)

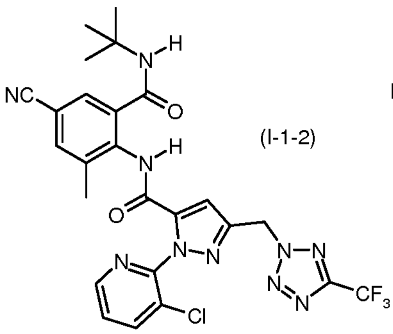
(I-1-2)

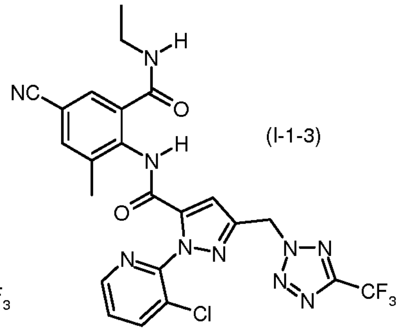
(I-1-3)

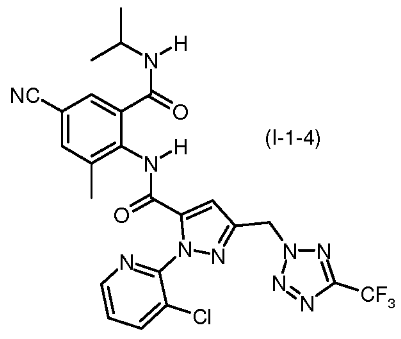
(I-1-4)

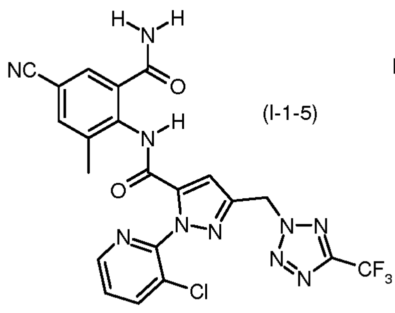
(I-1-5)

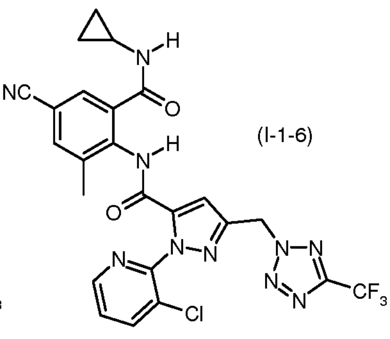
(I-1-6)

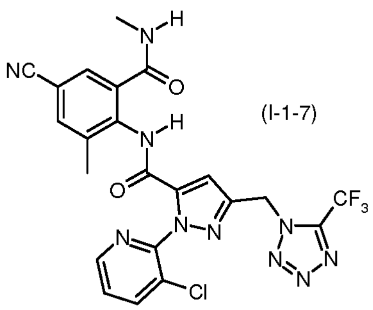
(I-1-7)

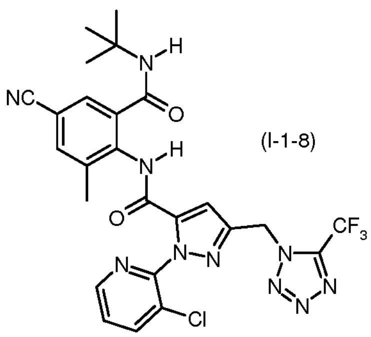
(I-1-8)

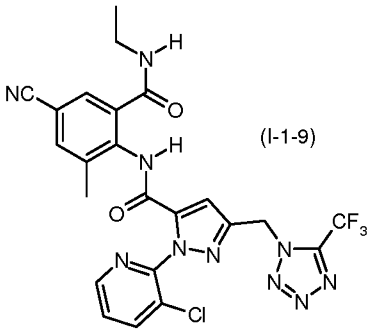
(I-1-9)

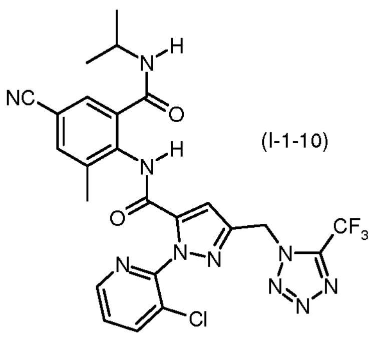
(I-1-10)

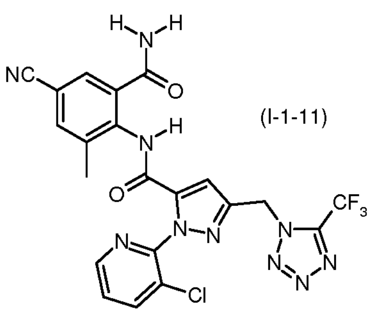
(I-1-11)

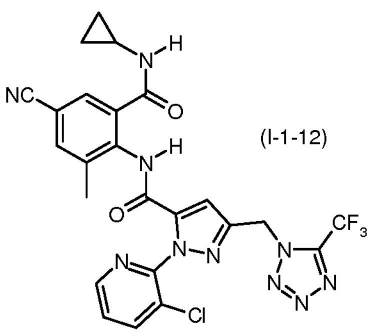
(I-1-12)

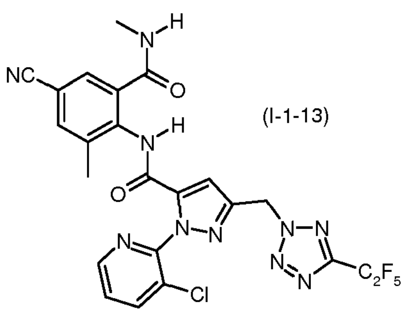
(I-1-13)

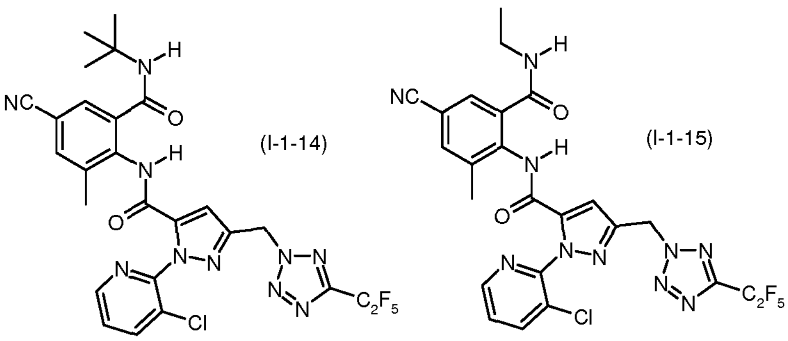
(I-1-14)

(I-1-15)

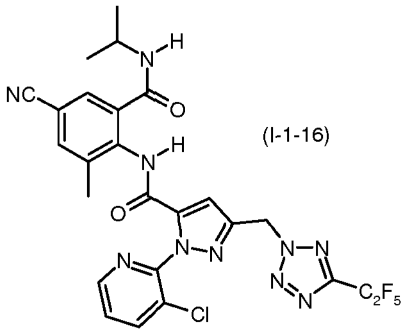

(I-1-16)

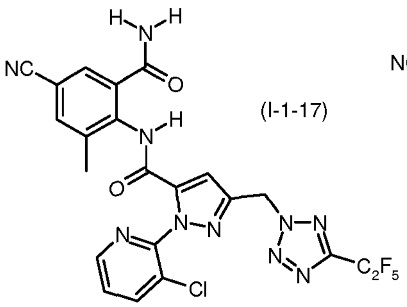

(I-1-17)

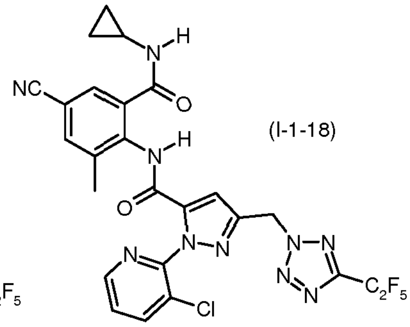

(I-1-18)

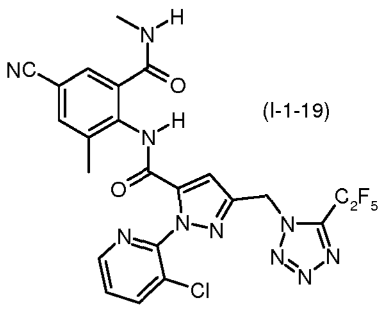

(I-1-19)

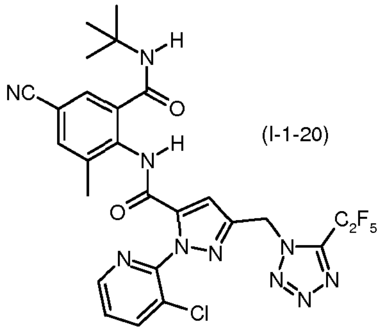

(I-1-20)

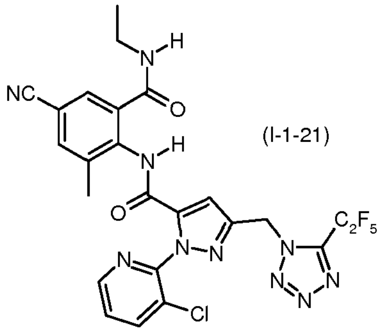

(I-1-21)

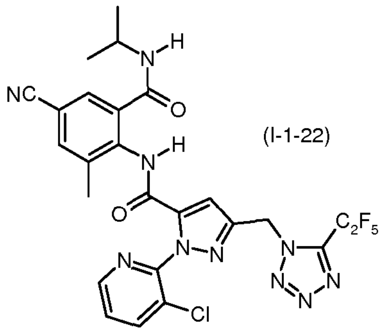

(I-1-22)

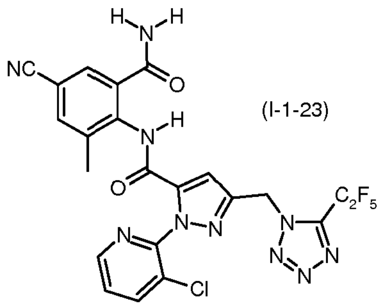

(I-1-23)

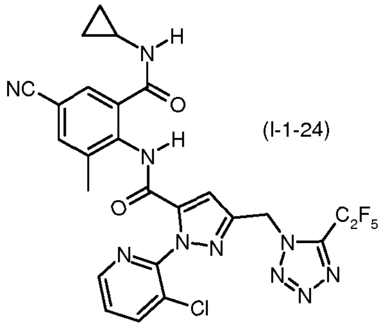

(I-1-24)

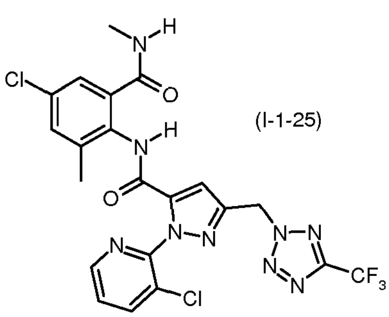

(I-1-25)

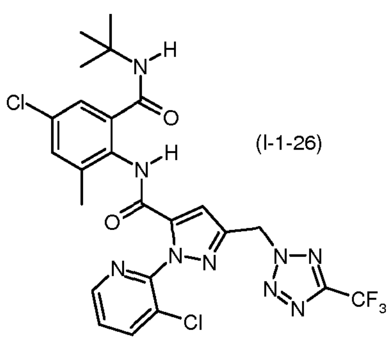

(I-1-26)

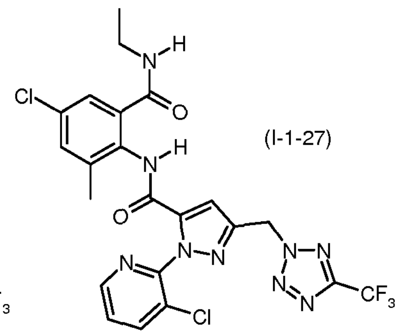

(I-1-27)

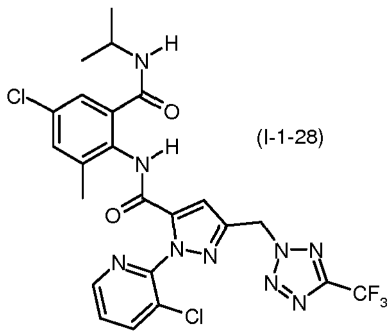
(I-1-28)

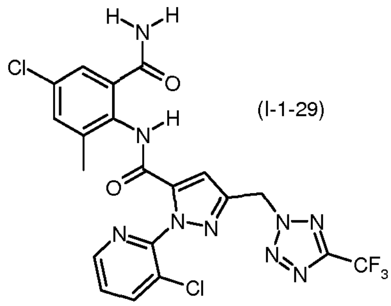
(I-1-29)

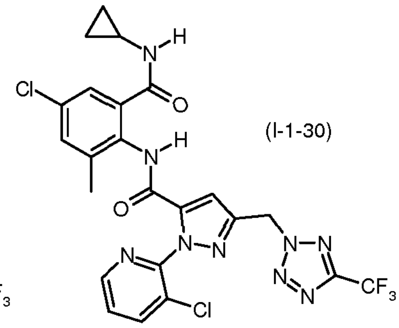
(I-1-30)

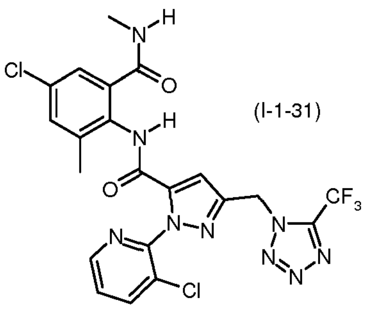
(I-1-31)

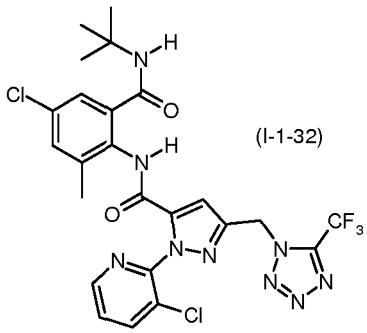
(I-1-32)

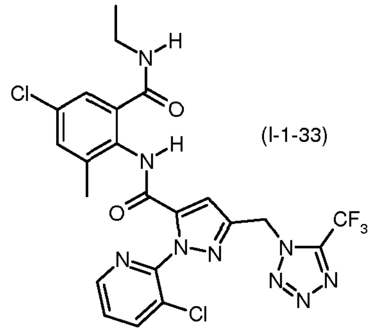
(I-1-33)

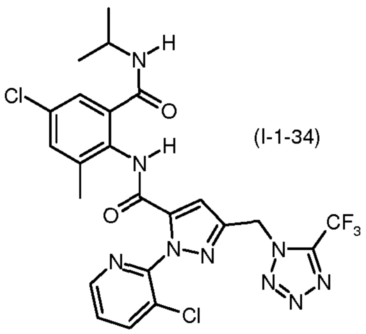
(I-1-34)

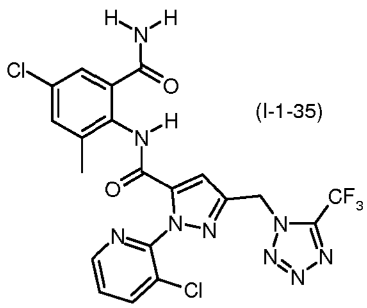
(I-1-35)

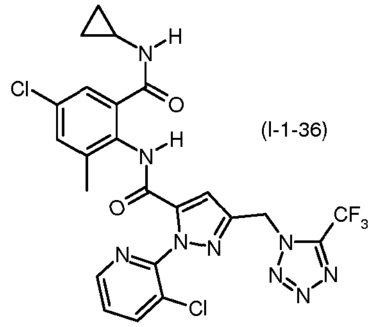
(I-1-36)

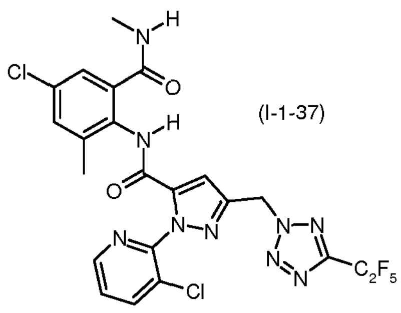
(I-1-37)

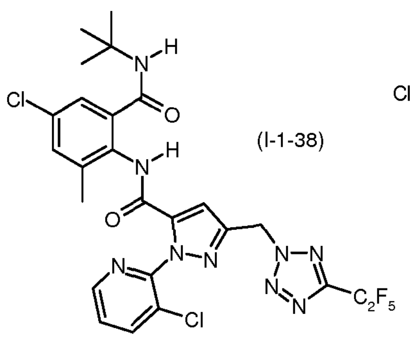
(I-1-38)

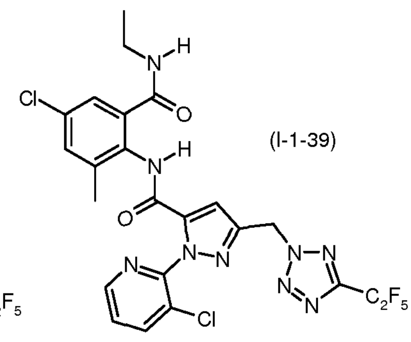
(I-1-39)

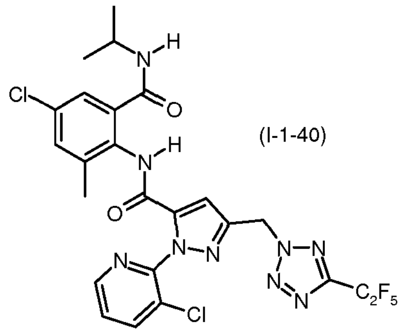
(I-1-40)

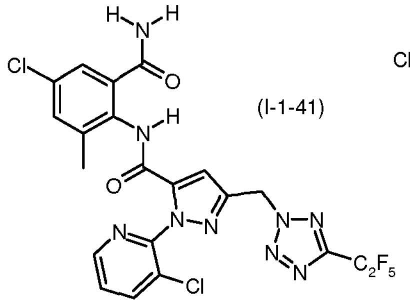
(I-1-41)

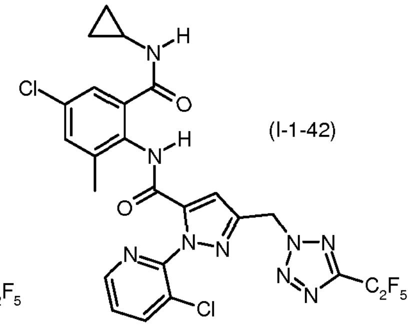
(I-1-42)

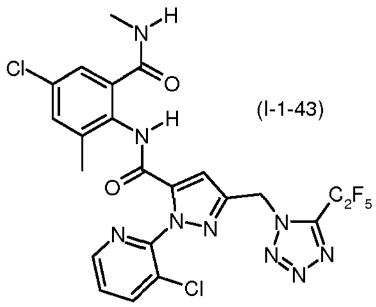
(I-1-43)

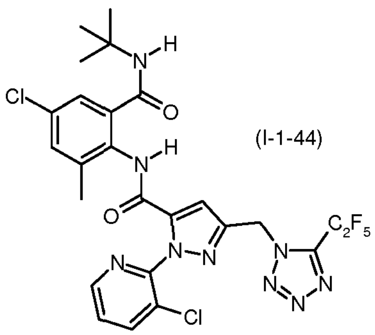
(I-1-44)

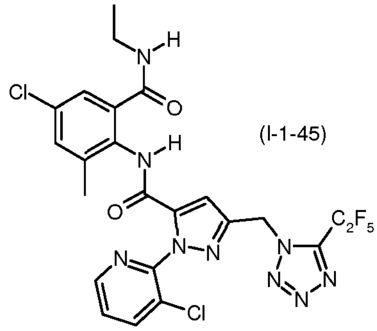
(I-1-45)

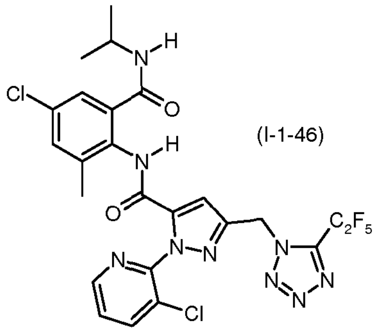
(I-1-46)

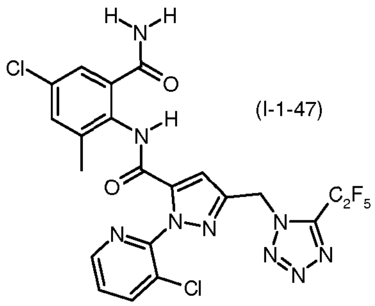
(I-1-47)

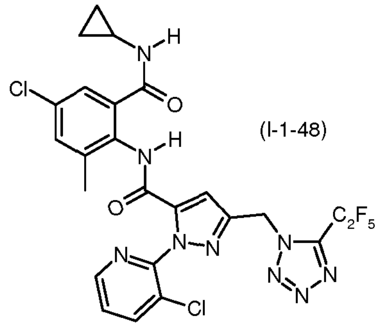
(I-1-48)

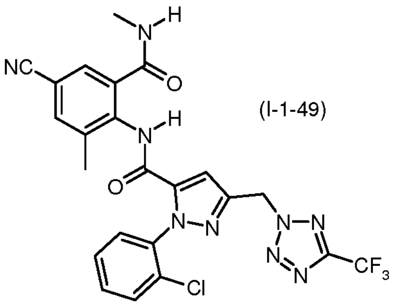
(I-1-49)

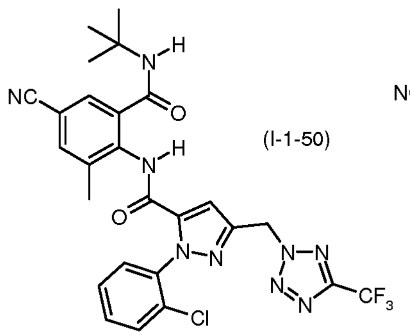
(I-1-50)

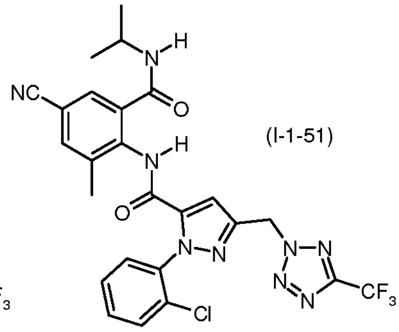
(I-1-51)

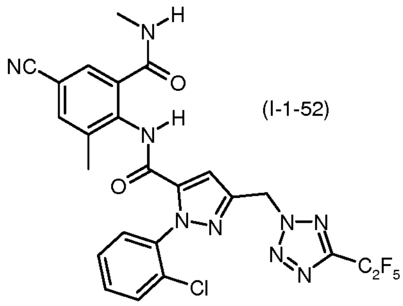
(I-1-52)

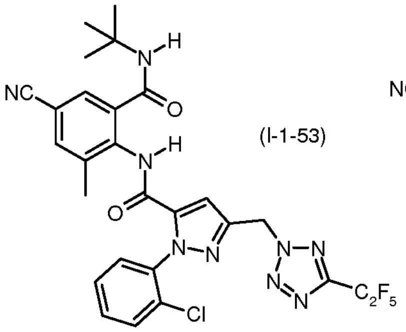
(I-1-53)

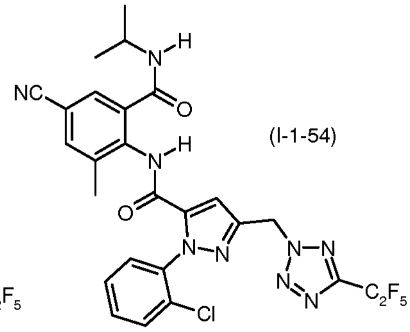
(I-1-54)

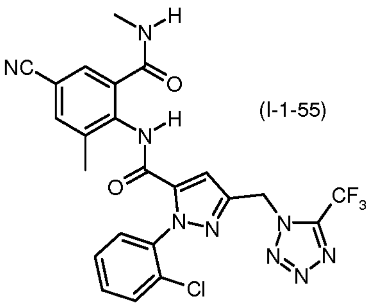
(I-1-55)

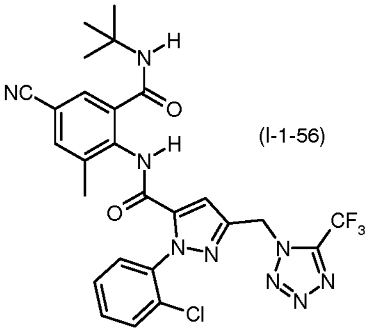
(I-1-56)

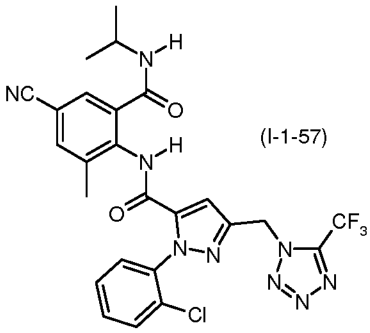
(I-1-57)

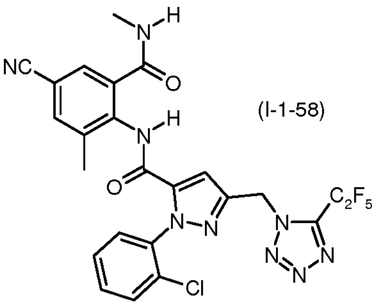
(I-1-58)

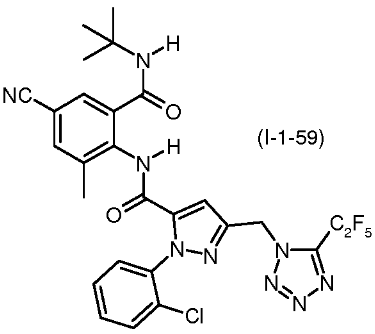
(I-1-59)

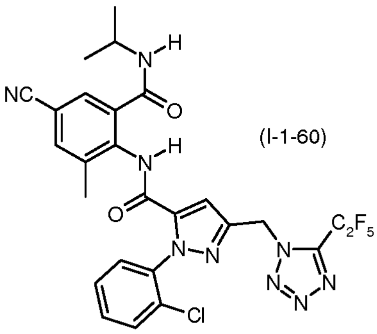
(I-1-60)

Weiterhin ganz besonders bevorzugt sind Wirkstoffkombinationen enthaltend die im folgenden angegebenen Mischungen aus Wirkstoffen der Formel (1-1-1) bis (1-1-60) und einen oder mehrere Wirkstoffe der Gruppe (II).

Diese Mischungen liegen bevorzugt in einem Mischungsverhältnis von 80:20 bis 99:1 vor. Beispielhaft erwähnt sei die Mischung I-1-1/I-1-7, wobei die Verbindung der Formel 1-1-1 zur Verbindung der Formel 1-1-7 in einem Mischungs verhältnis von 80:20 bis 99:1 vorliegt. Ebenfalls beispielhaft erwähnt sie die Mischung 1-1-2/ 1-1-8 wobei die Verbindung der Formel 1-1-2 zur Verbindung der Formel 1-1-8 in einem Mischungsverhältnis von 80:20 bis 99:1 vorliegt.

I-1-1-/1-1-7,
1-1-2/ 1-1-8,
1-1-3/1-1-9,
I-1-4/1-1-10,
I-1-5/1-1-11,
I-1-6/1-1-12,
I-1-13/I-1-1-19,
1-1-14/1-1-20,
I-1-15/1-1-21,
1-1-16/1-1-22,
I-1-17/I-1-23,
I-1-18/I-1-24,
1-1-25/1-1-3 l,
1-1-26/1-1-32,
I-1-27/ I-1-33,

1-1-28/1-1-34,
I-1-29/I-1-35,
I-1-30/I-1-36,
1-1-371-1-43,
1-1-38/1-1-44,
I-1-39/I-1-45,
I-1-40/I-1-46,
I-1-41/I-1-47,
I-1-42/I-1-48,
I-1-49/I-1-55,
I-1-50/I-1-56,
I-1-51/1-1-57,
I-1-52/I-1-58,
I-1-53/I-1-59,
I-1-54/I-1-60.

Die Wirkstoffkombinationen können darüber hinaus auch weitere fungizid, akarizid oder insektizid wirksame Zumischkomponenten enthalten.

Wenn die Wirkstoffe in den erfindungsgemäßen Wirkstoffkombinationen in bestimmten Gewichtsverhältnissen vorhanden sind, zeigt sich die verbesserte Wirkung. Jedoch können die Gewichtsverhältnisse der Wirkstoffe in den Wirkstoffkombinationen in einem relativ großen Bereich variiert werden. Im allgemeinen enthalten die erfindungsgemäßen Kombinationen Wirkstoffe der Formel (I) zum Mischpartner der Gruppe (II) im Verhältnis von 625:1 zu 1: 625; bevorzugt in den in der nachfolgenden Tabelle 1 angegeben bevorzugten und besonders bevorzugten Mischungsverhältnissen:

• die Mischungsverhältnisse basieren auf Gewichtsverhältnissen. Das Verhältnis ist zu verstehen als Wirkstoffe der Formel (I):Mischpartner bis Wirkstoffe der Formel (I):Mischpartner

| | Mischpartner | bevorzugtes Mischungsverhältnis | besonders bevorzugtes Mischungsverhältnis | ganz besonders bevorzugtes Mischungsverhältnis |
|---|---|---|---|---|
| 1. | Acrinathrin | 125:1 bis 1:125 | 25:1 bis 1:25 | 5:1 bis 1:5 |
| 2. | Alpha-Cypermethrin | 125:1 bis 1:125 | 25:1 bis 1:25 | 5:1 bis 1:5 |
| 3. | Betacyfluthrin | 125:1 bis 1:125 | 25:1 bis 1:25 | 5:1 bis 1:5 |
| 4. | Cyhalothrin | 125:1 bis 1:125 | 25:1 bis 1:25 | 5:1 bis 1:5 |
| 5. | Cypermethrin | 125:1 bis 1:125 | 25:1 bis 1:25 | 5:1 bis 1:5 |
| 6. | Deltamethrin | 125:1 bis 1:125 | 25:1 bis 1:25 | 5:1 bis 1:5 |
| 7. | Esfenvalerat | 125:1 bis 1:125 | 25:1 bis 1:25 | 5:1 bis 1:5 |
| 8. | Etofenprox | 125:1 bis 1:125 | 25:1 bis 1:25 | 5:1 bis 1:5 |
| 9. | Fenpropathrin | 125:1 bis 1:125 | 25:1 bis 1:25 | 5:1 bis 1:5 |
| 10. | Fenvalerat | 125:1 bis 1:125 | 25:1 bis 1:25 | 5:1 bis 1:5 |
| 11. | Flucythrinat | 125:1 bis 1:125 | 25:1 bis 1:25 | 5:1 bis 1:5 |
| 12.a | Lambda-Cyhalothrin | 125:1 bis 1:125 | 25:1 bis 1:25 | 5:1 bis 1:5 |
| 12.b | Gamma-Cyhalothrin | 125:1 bis 1:125 | 25:1 bis 1:25 | 5:1 bis 1:5 |
| 13. | Permethrin | 125:1 bis 1:125 | 25:1 bis 1:25 | 5:1 bis 1:5 |
| 14. | Tau-fluvalinat | 125:1 bis 1:125 | 25:1 bis 1:25 | 5:1 bis 1:5 |
| 15. | Transfluthrin | 125:1 bis 1:125 | 25:1 bis 1:25 | 5:1 bis 1:5 |
| 16. | Zeta-Cypermethrin | 125:1 bis 1:125 | 25:1 bis 1:25 | 5:1 bis 1:5 |
| 17. | Cyfluthrin | 125:1 bis 1:125 | 25:1 bis 1:25 | 5:1 bis 1:5 |
| 18. | Bifenthrin | 125:1 bis 1:125 | 25:1 bis 1:25 | 5:1 bis 1:5 |

(fortgesetzt)

| | Mischpartner | bevorzugtes Mischungsverhältnis | besonders bevorzugtes Mischungsverhältnis | ganz besonders bevorzugtes Mischungsverhältnis |
|---|---|---|---|---|
| 19. | Tefluthrin | 125:1 bis 1:125 | 25:1 bis 1:25 | 5:1 bis 1:5 |
| 20. | Eflusilanat | 125:1 bis 1:125 | 25:1 bis 1:25 | 5:1 bis 1:5 |
| 21. | Fubfenprox | 125:1 bis 1:125 | 25:1 bis 1:25 | 5:1 bis 1:5 |
| 22. | Pyrethrin | 125:1 bis 1:125 | 25:1 bis 1:25 | 5:1 bis 1:5 |
| 23. | Resmethrin | 125:1 bis 1:125 | 25:1 bis 1:25 | 5:1 bis 1:5 |
| 24. | Imidacloprid | 125:1 bis 1:125 | 25:1 bis 1:25 | 5:1 bis 1:5 |
| 25. | Acetamiprid | 125:1 bis 1:125 | 25:1 bis 1:25 | 5:1 bis 1:5 |
| 26. | Thiamethoxam | 125:1 bis 1:125 | 25:1 bis 1:25 | 5:1 bis 1:5 |
| 27. | Nitenpyram | 125:1 bis 1:125 | 25:1 bis 1:25 | 5:1 bis 1:5 |
| 28. | Thiacloprid | 125:1 bis 1:125 | 25:1 bis 1:25 | 5:1 bis 1:5 |
| 29. | Dinotefuran | 125:1 bis 1:125 | 25:1 bis 1:25 | 5:1 bis 1:5 |
| 30. | Clothianidin | 125:1 bis 1:125 | 25:1 bis 1:25 | 5:1 bis 1:5 |
| 31. | Imidaclothiz | 125:1 bis 1:125 | 25:1 bis 1:25 | 5:1 bis 1:5 |
| 32. | Chlorfluazuron | 125:1 bis 1:125 | 25:1 bis 1:25 | 5:1 bis 1:5 |
| 33. | Diflubenzuron | 125:1 bis 1:125 | 25:1 bis 1:25 | 5:1 bis 1:5 |
| 34. | Lufenuron | 125:1 bis 1:125 | 25:1 bis 1:25 | 5:1 bis 1:5 |
| 35. | Teflubenzuron | 125:1 bis 1:125 | 25:1 bis 1:25 | 5:1 bis 1:5 |
| 36. | Triflumuron | 125:1 bis 1:125 | 25:1 bis 1:25 | 5:1 bis 1:5 |
| 37. | Novaluron | 125:1 bis 1:125 | 25:1 bis 1:25 | 5:1 bis 1:5 |
| 38. | Flufenoxuron | 125:1 bis 1:125 | 25:1 bis 1:25 | 5:1 bis 1:5 |
| 39. | Hexaflumuron | 125:1 bis 1:125 | 25:1 bis 1:25 | 5:1 bis 1:5 |
| 40. | Bistrifluoron | 125:1 bis 1:125 | 25:1 bis 1:25 | 5:1 bis 1:5 |
| 41. | Noviflumuron | 125:1 bis 1:125 | 25:1 bis 1:25 | 5:1 bis 1:5 |
| 42. | Buprofezin | 625:1 bis 1:625 | 125:1 bis 1:125 | 25:1 bis 1:25 |
| 43. | Cyromazine | 125:1 bis 1:125 | 25:1 bis 1:25 | 5:1 bis 1:5 |
| 44. | Methoxyfenozide | 125:1 bis 1:125 | 25:1 bis 1:25 | 5:1 bis 1:5 |
| 45. | Tebufenozide | 125:1 bis 1:125 | 25:1 bis 1:25 | 5:1 bis 1:5 |
| 46. | Halofenozide | 125:1 bis 1:125 | 25:1 bis 1:25 | 5:1 bis 1:5 |
| 48. | Chromafenozide | 125:1 bis 1:125 | 25:1 bis 1:25 | 5:1 bis 1:5 |
| 49. | Endosulfan | 125:1 bis 1:125 | 25:1 bis 1:25 | 5:1 bis 1:5 |
| 50. | Fipronil | 125:1 bis 1:125 | 25:1 bis 1:25 | 5:1 bis 1:5 |
| 51. | Ethiprole | 125:1 bis 1:125 | 25:1 bis 1:25 | 5:1 bis 1:5 |
| 52. | Pyrafluprole | 125:1 bis 1:125 | 25:1 bis 1:25 | 5:1 bis 1:5 |
| 53. | Pyriprole | 125:1 bis 1:125 | 25:1 bis 1:25 | 5:1 bis 1:5 |
| 54. | Flubendiamide | 125:1 bis 1:125 | 25:1 bis 1:25 | 5:1 bis 1:5 |
| 55. | Chlorantraniliprole (Rynaxypyr) | 125:1 bis 1:125 | 25:1 bis 1:25 | 5:1 bis 1:5 |

(fortgesetzt)

| | Mischpartner | bevorzugtes Mischungsverhältnis | besonders bevorzugtes Mischungsverhältnis | ganz besonders bevorzugtes Mischungsverhältnis |
|---|---|---|---|---|
| 56. | Cyazypyr | 125:1 bis 1:125 | 25:1 bis 1:25 | 5:1 bis 1:5 |
| 57. | Emamectin | 125:1 bis 1:125 | 25:1 bis 1:25 | 5:1 bis 1:5 |
| 58. | Emamectin benzoate | 125:1 bis 1:125 | 25:1 bis 1:25 | 5:1 bis 1:5 |
| 59. | Abamectin | 125:1 bis 1:125 | 25:1 bis 1:25 | 5:1 bis 1:5 |
| 60. | Ivermectin | 125:1 bis 1:125 | 25:1 bis 1:25 | 5:1 bis 1:5 |
| 61. | Milbemectin | 125:1 bis 1:125 | 25:1 bis 1:25 | 5:1 bis 1:5 |
| 62. | Lepimectin | 125:1 bis 1:125 | 25:1 bis 1:25 | 5:1 bis 1:5 |
| 63. | Tebufenpyrad | 125:1 bis 1:125 | 25:1 bis 1:25 | 5:1 bis 1:5 |
| 64. | Fenpyroximat | 125:1 bis 1:125 | 25:1 bis 1:25 | 5:1 bis 1:5 |
| 65. | Pyridaben | 125:1 bis 1:125 | 25:1 bis 1:25 | 5:1 bis 1:5 |
| 66. | Fenazaquin | 125:1 bis 1:125 | 25:1 bis 1:25 | 5:1 bis 1:5 |
| 67. | Pyrimidifen | 125:1 bis 1:125 | 25:1 bis 1:25 | 5:1 bis 1:5 |
| 68. | Tolfenpyrad | 125:1 bis 1:125 | 25:1 bis 1:25 | 5:1 bis 1:5 |
| 69. | Dicofol | 125:1 bis 1:125 | 25:1 bis 1:25 | 5:1 bis 1:5 |
| 70. | Cyenopyrafen | 125:1 bis 1:125 | 25:1 bis 1:25 | 5:1 bis 1:5 |
| 71. | Cyflumetofen | 125:1 bis 1:125 | 25:1 bis 1:25 | 5:1 bis 1:5 |
| 72. | Acequinocyl | 125:1 bis 1:125 | 25:1 bis 1:25 | 5:1 bis 1:5 |
| 73. | Fluacrypyrin | 125:1 bis 1:125 | 25:1 bis 1:25 | 5:1 bis 1:5 |
| 74. | Bifenazate | 125:1 bis 1:125 | 25:1 bis 1:25 | 5:1 bis 1:5 |
| 75. | Diafenthiuron | 125:1 bis 1:125 | 25:1 bis 1:25 | 5:1 bis 1:5 |
| 76. | Etoxazole | 125:1 bis 1:125 | 25:1 bis 1:25 | 5:1 bis 1:5 |
| 77. | Clofentezine | 125:1 bis 1:125 | 25:1 bis 1:25 | 5:1 bis 1:5 |
| 78. | Spinosad | 125:1 bis 1:125 | 25:1 bis 1:25 | 5:1 bis 1:5 |
| 79. | Triarathen | 125:1 bis 1:125 | 25:1 bis 1:25 | 5:1 bis 1:5 |
| 80. | Tetradifon | 125:1 bis 1:125 | 25:1 bis 1:25 | 5:1 bis 1:5 |
| 81. | Propargit | 125:1 bis 1:125 | 25:1 bis 1:25 | 5:1 bis 1:5 |
| 82. | Hexythiazox | 125:1 bis 1:125 | 25:1 bis 1:25 | 5:1 bis 1:5 |
| 83. | Bromopropylat | 125:1 bis 1:125 | 25:1 bis 1:25 | 5:1 bis 1:5 |
| 84. | Chinomethionat | 125:1 bis 1:125 | 25:1 bis 1:25 | 5:1 bis 1:5 |
| 85. | Amitraz | 125:1 bis 1:125 | 25:1 bis 1:25 | 5:1 bis 1:5 |
| 86. | Pyrifluquinazone | 125:1 bis 1:125 | 25:1 bis 1:25 | 5:1 bis 1:5 |
| 87. | Pymetrozine | 125:1 bis 1:125 | 25:1 bis 1:25 | 5:1 bis 1:5 |
| 88. | Flonicamid | 125:1 bis 1:125 | 25:1 bis 1:25 | 5:1 bis 1:5 |
| 89. | Pyriproxyfen | 125:1 bis 1:125 | 25:1 bis 1:25 | 5:1 bis 1:5 |
| 90. | Diofenolan | 125:1 bis 1:125 | 25:1 bis 1:25 | 5:1 bis 1:5 |
| 91. | Chlorfenapyr | 125:1 bis 1:125 | 25:1 bis 1:25 | 5:1 bis 1:5 |

(fortgesetzt)

| | Mischpartner | bevorzugtes Mischungsverhältnis | besonders bevorzugtes Mischungsverhältnis | ganz besonders bevorzugtes Mischungsverhältnis |
|---|---|---|---|---|
| 92. | Metaflumizone | 125:1 bis 1:125 | 25:1 bis 1:25 | 5:1 bis 1:5 |
| 93. | Indoxacarb | 125:1 bis 1:125 | 25:1 bis 1:25 | 5:1 bis 1:5 |
| 94. | Chlorpyrifos | 125:1 bis 1:125 | 25:1 bis 1:25 | 5:1 bis 1:5 |
| 95. | Spirodiclofen | 125:1 bis 1:125 | 25:1 bis 1:25 | 5:1 bis 1:5 |
| 96. | Spiromesifen | 125:1 bis 1:125 | 25:1 bis 1:25 | 5:1 bis 1:5 |
| 97. | Spirotetramat | 125:1 bis 1:125 | 25:1 bis 1:25 | 5:1 bis 1:5 |
| 98. | Pyridalyl | 125:1 bis 1:125 | 25:1 bis 1:25 | 5:1 bis 1:5 |
| 99. | Spinetoram | 125:1 bis 1:125 | 25:1 bis 1:25 | 5:1 bis 1:5 |
| 100. | Acephate | 125:1 bis 1:125 | 25:1 bis 1:25 | 5:1 bis 1:5 |
| 101. | Triazophos | 125:1 bis 1:125 | 25:1 bis 1:25 | 5:1 bis 1:5 |
| 102. | Profenofos | 125:1 bis 1:125 | 25:1 bis 1:25 | 5:1 bis 1:5 |
| 103. | Fenamiphos | 125:1 bis 1:125 | 25:1 bis 1:25 | 5:1 bis 1:5 |
| 104. | 4-{[(6-Chlorpyrid-3-yl)methyl](2,2-difluorethyl)amino}furan -2(5H)-on | 125:1 bis 1:125 | 25:1 bis 1:25 | 5:1 bis 1:5 |
| 105. | Cadusaphos | 125:1 bis 1:125 | 25:1 bis 1:25 | 5:1 bis 1:5 |
| 106. | Carbaryl | 125:1 bis 1:125 | 25:1 bis 1:25 | 5:1 bis 1:5 |
| 107. | Carbofuran | 125:1 bis 1:125 | 25:1 bis 1:25 | 5:1 bis 1:5 |
| 108. | Ethoprophos | 125:1 bis 1:125 | 25:1 bis 1:25 | 5:1 bis 1:5 |
| 109. | Thiodicarb | 125:1 bis 1:125 | 25:1 bis 1:25 | 5:1 bis 1:5 |
| 110. | Aldicarb | 125:1 bis 1:125 | 25:1 bis 1:25 | 5:1 bis 1:5 |
| 111. | Metamidophos | 125:1 bis 1:125 | 25:1 bis 1:25 | 5:1 bis 1:5 |
| 112. | Methiocarb | 125:1 bis 1:125 | 25:1 bis 1:25 | 5:1 bis 1:5 |
| 113. | Sulfoxaflor | 125:1 bis 1:125 | 25:1 bis 1:25 | 5:1 bis 1:5 |
| 114. | Imicyafos | 125:1 bis 1:125 | 25:1 bis 1:25 | 5:1 bis 1:5 |
| 115. | Fluensulfone | 125:1 bis 1:125 | 25:1 bis 1:25 | 5:1 bis 1:5 |
| 116. | Bacillus firmus 1-1582 | 500:1 bis 1:500 | 125:1 bis 1:125 | 25:1 bis 1:25 |
| 117. | 11-(4-Chlor-2,6-dimethylphenyl)-12-hydroxy-1,4-dioxa-9-azadispiro[4.2.4.2]tetra dec-11-en-10-on | 125:1 bis 1:125 | 25:1 bis 1:25 | 5:1 bis 1:5 |
| 118. | 2-{6-[2-(5-Fluorpyridin-3-yl)-1,3-thiazol-5-yl]pyridin-2-yl}pyrimidin | 125:1 bis 1:125 | 25:1 bis 1:25 | 5:1 bis 1:5 |

Die erfindungsgemäßen Wirkstoffkombinationen eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und

resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Halotydeus destructor, Hemitarsonemus spp., Hyalomma spp., Ixodes spp., Latrodectus mactans, Metatetranychus spp., Nuphersa spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acalymma vittatum, Acanthoscelides obtectus, Adoretus spp., Agelastica alni, Agriotes spp., Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptorhynchus lapathi, Cylindrocopturus spp., Dermestes spp., Diabrotica spp., Dichocrocis spp., Diloboderus spp., Epilachna spp., Epitrix spp., Faustinus spp., Gibbium psylloides, Hellula undalis, Heteronychus arator, Heteronyx spp., Hylamorpha elegans, Hylotrupes bajulus, Hypera postica, Hypothenemus spp., Lachnosterna consanguinea, Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllotreta spp., Popillia japonica, Premnotrypes spp., Psylliodes spp., Ptinus spp., Rhizobius ventralis, Rhizopertha dominica, Sitophilus spp., Sphenophorus spp., Sternechus spp., Symphyletes spp., Tanymecus spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Diplopoda z.B. Blaniulus guttulatus.

Aus der Ordnung der Diptera z.B. Aedes spp., Agromyza spp., Anastrepha spp., Anopheles spp.,

Asphondylia spp., Bactrocera spp., Bibio hortulanus, Calliphora erythrocephala, Ceratitis capitata, Chironomus spp., Chrysomyia spp., Cochliomyia spp., Contarinia spp., Cordylobia anthropophaga, Culex spp., Cuterebra spp., Dacus oleae, Dasyneura spp., Delia spp., Dermatobia hominis, Drosophila spp., Echinocnemus spp., Fannia spp., Gastrophilus spp., Hydrellia spp., Hylemyia spp., Hyppobosca spp., Hypoderma spp., Liriomyza spp.. Lucilia spp., Musca spp., Nezara spp., Oestrus spp., Oscinella frit, Pegomyia spp., Phorbia spp., Prodiplosis spp., Psila rosae, Rhagoletis spp., Stomoxys spp., Tabanus spp., Tannia spp., Tetanops spp., Tipula spp..

Aus der Klasse der Gastropoda z.B. Arion spp., Biomphalaria spp., Bulinus spp., Deroceras spp., Galba spp., Lymnaea spp., Oncomelania spp., Pomacea spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.

Aus der Ordnung der Homoptera z.B. Acyrthosipon spp., Acrogonia spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia

spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Ferrisia spp., Geococcus coffeae, Hieroglyphus spp., Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes spp., Trioza spp., Typhlocyba spp., Unaspis spp., Viteus vitifolii, Zygina spp..

Aus der Ordnung der Hymenoptera z.B. Athalia spp., Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Acromyrmex spp., Atta spp., Cornitermes cumulans, Microtermes obesi, Odontotermes spp., Reticulitermes spp,

Aus der Ordnung der Lepidoptera z.B. Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia kuehniella, Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamestra brassicae, Mocis spp., Mythimna separata, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plusia spp., Plutella xylostella, Prays spp., Prodenia spp., Protoparce spp., Pseudaletia spp., Pseudoplusia includens, Pyrausta nubilalis, Rachiplusia nu, Schoenobius spp., Scirpophaga spp., Scotia segetum, Sesamia spp., Sparganothis spp., Spodoptera spp., Stathmopoda spp., Stomopteryx subsecivella, Synanthedon spp., Tecia solanivora, Thermesia gemmatalis, Tinea pellionella, Tineola bisselliella, Tortrix spp., Trichoplusia spp., Tuta absoluta, Virachola spp..

Aus der Ordnung der Orthoptera z.B. Acheta domesticus, Blatta orientalis, Blattella germanica, Dichroplus spp., Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella spp..

Aus der Ordnung der Thysanoptera z.B. Anaphothrips obscurus, Baliothrips biformis, Drepanothris reuteri, Enneothrips flavens, Frankliniella spp., Heliothrips spp., Hercinothrips femoralis, Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Aphelenchoides spp., Bursaphelenchus spp., Ditylenchus spp., Globodera spp., Heterodera spp., Longidorus spp., Meloidogyne spp., Pratylenchus spp., Radopholus similis, Trichodorus spp., Tylenchulus semipenetrans, Xiphinema spp..

Die Wirkstoffkombinationen können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol,

Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffkombinationen können in handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Auch eine Mischung mit anderen bekannten Wirkstoffen, wie Herbiziden oder mit Düngemitteln und Wachstumsregulatoren ist möglich.

Die erfindungsgemäßen Wirkstoffkombinationen können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Erfindungsgemäß können alle Planzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft, Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhiozome, Ableger und Samen.

Die erfindungsgemäße Behandlung der Pflanzen und Pflanzenteile mit den Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Verdampfen, Angießen, Vernebeln, Streuen, Aufstreichen und bei Vermehrungsmaterial, insbesondere bei Samen, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer

weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit der erfindungsgemäßen Wirkstoffmischung behandelt werden. Die bei den Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Mischungen.

Die gute insektizide und akarizide Wirkung der erfindungsgemäßen Wirkstoffkombinationen geht aus den nachfolgenden Beispielen hervor. Während die einzelnen Wirkstoffe in der Wirkung Schwächen aufweisen, zeigen die Kombinationen eine Wirkung, die über eine einfache Wirkungssummierung hinausgeht.

Ein synergistischer Effekt liegt bei Insektiziden und Akariziden immer dann vor, wenn die Wirkung der Wirkstoffkombinationen größer ist als die Summe der Wirkungen der einzeln applizierten Wirkstoffe.

## Anwendungsbeispiele

Die zu erwartende Wirkung für eine gegebene Kombination zweier Wirkstoffe kann nach S.R. Colby, Weeds 15 (1967), 20-22 wie folgt berechnet werden:

Wenn

X den Abtötungsgrad, ausgedrückt in % der unbehandelten Kontrolle, beim Einsatz des Wirkstoffes A in einer Aufwandmenge von m g/ha oder in einer Konzentration von m ppm bedeutet,

Y den Abtötungsgrad, ausgedrückt in % der unbehandelten Kontrolle, beim Einsatz des Wirkstoffes B in einer Aufwandmenge von n g/ha oder in einer Konzentration von n ppm bedeutet und

E den Abtötungsgrad, ausgedrückt in % der unbehandelten Kontrolle, beim Einsatz der Wirkstoffe A und B in Aufwandmengen von m und n g/ha oder in einer Konzentration von m und n ppm bedeutet,

dann ist

$$E = X + Y - \frac{X \cdot Y}{100}.$$

Ist der tatsächliche Abtötungsgrad größer als berechnet, so ist die Kombination in ihrer Abtötung überadditiv, d.h. es liegt ein synergistischer Effekt vor. In diesem Fall muss der tatsächlich beobachtete Abtötungsgrad größer sein als der aus der oben angeführten Formel errechnete Wert für den erwarteten Abtötungsgrad (E).

Beispiel **A**

**Myzus persicae -Test**

| | |
|---|---|
| Lösungsmittel: | 78 Gewichtsteile Aceton<br>1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (*Brassica oleracea*), die stark von der Grünen Pfirsichblattlaus (*Myzus persicae*) befallen sind, werden durch Spritzen mit der Wirkstoffzubereitung in der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden. Die ermittelten Abtötungswerte verrechnet man nach der Colby-Formel.

Bei diesem Test zeigen z. B. die folgenden Wirkstoffkombinationen gemäß vorliegender Anmeldung eine synergistisch verstärkte Wirksamkeit im Vergleich zu den einzeln angewendeten Wirkstoffen:

Tabelle A - 1: **Myzus persicae - Test**

| Wirkstoff | Konzentration in g/ha | Abtötung in % nach $1^d$ |
|---|---|---|
| **Verbindung (I-1-2)/Verbindung (I-1-8)*** ** | 4<br>0,8<br>0,16<br>0,032 | 0<br>0<br>0<br>0 |
| **Verbindung (I-1-1)/Verbindung (I-1-7)*** ** | 4<br>0,8<br>0,16<br>0,032 | 80<br>0<br>0<br>0 |
| **Abamectin** | 0,16 | 0 |
| **Verbindung (I-1-1)/Verbindung (I-1-7)*** + Abamectin (5 : 1)**<br>erfindungsgemäß | **0,8 + 0,16** | gef.* ber.**<br>**70 0** |
| **Acephate** | 100 | 0 |
| **Verbindung (I-1-2)/Verbindung (I-1-8)*** + Acephate (1 : 25)**<br>erfindungsgemäß | **4 + 100** | gef.* ber.**<br>**80 0** |
| **Acrinathrin** | 0,8 | 0 |
| **Verbindung (1-1-2)Verbindung (I-1-8)*** + Acrinathrin (1 :25)** | **0,032 + 0,8** | gef.* ber.**<br>**90 0** |
| erfindungsgemäß | | |
| **Verbindung (I-1-1)/Verbindung (I-1-7)*** + Acrinathrin (1 : 25)**<br>erfindungsgemäß | **0,032 + 0,8** | gef.* ber.**<br>**80 0** |
| **Alpha-Cypermethrin** | 0,16 | 70 |

(fortgesetzt)

| Wirkstoff | Konzentration in g/ha | Abtötung in % nach 1d | |
|---|---|---|---|
| **Verbindung (I-1-2)/Verbindung (I-1-8)*** + Alpha-Cypermethrin (1 : 1)** erfindungsgemäß | **0,16 + 0,16** | **gef.*** **90** | **ber.**** **70** |
| **Bacillus firmus strain I-1582** | 250 | 0 | |
| **Verbindung (I-1-1)/Verbindung (I-1-7)*** + Bacillus firmus I-1582 (1 : 312,5)** erfindungsgemäß | **0,8 + 250** | **gef.*** **100** | **ber.**** **80** |
| **Bifenthrin** | 0,8 | 80 | |
| **Verbindung (I-1-1)/Verbindung (I-1-7)*** + Bifenthrin (1 : 1)** erfindungsgemäß | 0,8 + **0,8** | **gef.*** **100** | **ber.**** **80** |
| β-**Cyfluthrin** | 0,16<br>0,032 | 70<br>0 | |
| **Verbindung (I-1-2)/Verbindung (I-1-8)*** + β - Cyfluthrin (5 : 1)** erfindungsgemäß | **0,16 + 0,032** | **gef.*** **70** | **ber.**** **0** |
| **Verbindung (I-1-1)/Verbindung (I-1-7)*** + β - Cyfluthrin** (5 : 1) erfindungsgemäß | **0,8 + 0,16** | **gef.*** **90** | **ber.**** **70** |
| **Buprofezin** | 500 | 0 | |
| **Verbindung (I-1-1)/Verbindung (I-1-7)*** + Buprofezin (1 : 625)** erfindungsgemäß | **0,8 + 500** | **gef.*** **70** | **ber.**** **0** |
| **Cadusaphos** | 20 | 0 | |

(fortgesetzt)

| **Wirkstoff** | **Konzentration in g/ha** | **Abtötung in % nach 1[d]** | |
|---|---|---|---|
| | | **gef.*** | **ber.**** |
| **Verbindung (I-1-2)/Verbindung (I-1-8)*** + Cadusaphos (1 : 25)** erfindungsgemäß | **0,8 + 20** | **90** | **0** |
| **Verbindung (I-1-1)/Verbindung (I-1-7)*** + Cadusaphos (1 : 25)** erfindungsgemäß | **0,8 + 20** | **90** | **0** |
| **Carbaryl** | 500 | 0 | |
| **Verbindung (I-1-2)/Verbindung (I-1-8)*** + Carbaryl (1 : 125)** erfindungsgemäß | **4 + 500** | **90** | **0** |
| **Carbofuran** | 20 | 70 | |
| **Verbindung (I-1-2)/Verbindung (I-1-8)*** + Carbofuran (1 : 25)** erfindungsgemäß | **0,8 + 20** | 90 | **70** |
| **Chlorpyrifos** | 4 | 80 | |
| **Verbindung (I-1-2)/Verbindung (I-1-8)*** + Chlorpyrifos (1 : 25)** erfindungsgemäß | **0,16 + 4** | **100** | **80** |
| **Clothianidin** | 20 | 70 | |
| **Verbindung (I-1-2)/Verbindung (I-1-8)*** + Clothianidin (1 : 5)** erfindungsgemäß | **4+20** | **90** | **70** |
| **Cypermethrin** | 4 | 80 | |

(fortgesetzt)

| Wirkstoff | Konzentration in g/ha | Abtötung in % nach 1$^{d}$ | |
|---|---|---|---|
| **Verbindung (I-1-1)/Verbindung (I-1-7)*** + Cypermethrin (1 : 5)** erfindungsgemäß | **0,8 + 4** | **gef.*** **100** | **ber.**** **80** |
| **Deltamethrin** | 0,8 | 0 | |
| **Verbindung (1-1-2)Verbindung (I-1-8)*** + Deltamethrin (1 : 1)** erfindungsgemäß | **0,8 + 0,8** | **gef.*** **100** | **ber.**** **0** |
| **Verbindung (I-1-1)/Verbindung (I-1-7)*** + Deltamethrin (1 : 1)** erfindungsgemäß | **0,8 + 0,8** | **gef.*** **80** | **ber.**** **0** |
| **Diafenthiuron** | 100 | 0 | |
| **Verbindung (I-1-1)/Verbindung (I-1-7)*** + Diafenthiuron (1 : 125)** erfindungsgemäß | **0,8 + 100** | **gef.*** **70** | **ber.**** **0** |
| **Emamectin-benzoate** | 0,8<br>0,16 | 0<br>0 | |
| **Verbindung (I-1-2)/Verbindung (I-1-8)*** + Emamectin-benzoate (5 : 1)** erfindungsgemäß | **4 + 0,8** | **gef.*** **70** | **ber.**** **0** |
| **Verbindung (I-1-1)/Verbindung (I-1-7)*** + Emamectin-benzoate (5 : 1)** erfindungsgemäß | **0,8 + 0,16** | **gef.*** **70** | **ber.**** **0** |
| **Ethiprole** | 4 | 0 | |
| **Verbindung (I-1-2)/Verbindung (I-1-8)*** + Ethiprole (1 : 5)** | **0,8 + 4** | **gef.*** **80** | **ber.**** **0** |

(fortgesetzt)

| Wirkstoff | Konzentration in g/ha | Abtötung in % nach 1d | |
|---|---|---|---|
| erfindungsgemäß | | | |
| **Verbindung (I-1-1)/Verbindung (I-1-7)*** + Ethiprole (1 : 5)**<br>erfindungsgemäß | **0,8 + 4** | gef.*<br>**70** | ber.**<br>**0** |
| **Flonicamid** | 20 | 0 | |
| **Verbindung (1-1-2)Verbindung (I-1-8)*** + Flonicamid (1 : 25)**<br>erfindungsgemäß | **0,8 + 20** | gef.*<br>**80** | ber.**<br>**0** |
| **Verbindung (I-1-1)Nerbindung (I-1-7)*** ** | | gef.* | ber.** |
| + **Flonicamid (1 : 25)**<br>erfindungsgemäß | **0,8 + 20** | **70** | **0** |
| **Gamma-Cyhalothrin** | 0,032 | 0 | |
| **Verbindung (I-1-1)/Verbindung (I-1-7)*** + Gamma-Cyhalothrin** (5 : 1)<br>erfindungsgemäß | **0,16 + 0,032** | gef.*<br>**70** | ber.**<br>**0** |
| **Imidacloprid** | 0,8 | 0 | |
| **Verbindung (I-1-2)/Verbindung (I-1-8)*** + Imidacloprid (1 : 25)**<br>erfindungsgemäß | **0,032 + 0,8** | gef.*<br>**70** | ber.**<br>**0** |
| **Verbindung (I-1-1)/Verbindung (I-1-7)*** + Imidacloprid (1 : 25)**<br>erfindungsgemäß | **0,032 + 0,8** | gef.*<br>**70** | ber.**<br>**0** |
| **Indoxacarb** | 20 | 0 | |
| **Verbindung (1-1-2)Verbindung (I-1-8)*** + Indoxacarb (1 : 25)** | **0,8 + 20** | gef.*<br>70 | ber.**<br>0 |

(fortgesetzt)

| Wirkstoff | Konzentration in g/ha | Abtötung in % nach 1$^d$ | |
|---|---|---|---|
| erfindungsgemäß | | | |
| **Verbindung (I-1-1)/Verbindung (I-1-7)*** + Indoxacarb (1 : 25)**<br>erfindungsgemäß | **0,8 + 20** | **gef.***<br>**80** | **ber.****<br>**0** |
| **L-Cyhalothrin** | 0,16 | 0 | |
| **Verbindung (I-1-2)/Verbindung (I-1-8)*** + L-Cyhalothrin (1 : 1)**<br>erfindungsgemäß | **0,16 + 0,16** | **gef.***<br>**80** | **ber.****<br>**0** |
| **Verbindung (I-1-1)/Verbindung (I-1-7)*** + L-Cyhalothrin (1 : 1)**<br>erfindungsgemäß | **0,16 + 0,16** | **gef.***<br>**90** | **ber.****<br>**0** |
| **Metaflumizone** | 20 | 0 | |
| **Verbindung (I-1-2)/Verbindung (I-1-8)*** + Metaflumizone (1 : 25)**<br>erfindungsgemäß | **0,8 + 20** | **gef.***<br>**70** | **ber.****<br>**0** |
| **Verbindung (I-1-1)/Verbindung (I-1-7)*** + Metaflumizone(1 : 25)**<br>erfindungsgemäß | **0,8 + 20** | **gef.***<br>**70** | **ber.****<br>**0** |
| **Methamidophos** | 100<br>20 | 80<br>0 | |
| **Verbindung (1-1-2)Verbindung (I-1-8)*** + Methamidophos (1 : 25)**<br>erfindungsgemäß | **4 + 100** | **gef.***<br>**100** | **ber.****<br>**80** |
| **Verbindung (I-1-1)/Verbindung (I-1-7)*** + Methamidophos (1 : 25)**<br>erfindungsgemäß | **0,8 + 20** | **gef.***<br>**90** | **ber.****<br>**0** |

(fortgesetzt)

| Wirkstoff | Konzentration in g/ha | Abtötung in % nach 1[d] | |
|---|---|---|---|
| **Methiocarb** | 20 | 0 | |
| **Verbindung (I-1-2)/Verbindung (I-1-8)***** | | gef.* | ber.** |
| **+ Methiocarb (1 : 25)**<br>erfindungsgemäß | **0,8 + 20** | **70** | **0** |
| **Verbindung (I-1-1)/Verbindung (I-1-7)*** + Methiocarb (1 : 25)**<br>erfindungsgemäß | **0,8 + 20** | gef.*<br>**70** | ber.**<br>**0** |
| **Milbemectin** | 0,8 | 0 | |
| **Verbindung (I-1-1)/Verbindung (I-1-7)*** + Milbemectin (1 : 1)**<br>erfindungsgemäß | **0,8 + 0,8** | gef.*<br>**70** | ber.**<br>**0** |
| **Pyridalyl** | 4 | 0 | |
| **Verbindung (I-1-2)/Verbindung (I-1-8)*** + Pyridalyl (1 : 5)**<br>erfindungsgemäß | **0,8 + 4** | gef.*<br>**70** | ber.**<br>**0** |
| **Spinetoram** | 4 | 0 | |
| **Verbindung (I-1-2)/Verbindung (I-1-8)*** + Spinetoram (1 : 1)**<br>erfindungsgemäß | **4+4** | gef.*<br>**70** | ber.**<br>**0** |
| **Spirodiclofen** | 100<br>20 | 0<br>0 | |
| **Verbindung (I-1-2)/Verbindung (I-1-8)*** + Spirodiclofen (1 : 25)**<br>erfindungsgemäß | 4 + **100** | gef.*<br>**70** | ber.**<br>**0** |

(fortgesetzt)

| Wirkstoff | Konzentration in g/ha | Abtötung in % nach 1[d] | |
|---|---|---|---|
| **Verbindung (I-1-1)/Verbindung (I-1-7)*** + Spirodiclofen (1 : 25)** erfindungsgemäß | **0,8 + 20** | **gef.*** **70** | **ber.**** **0** |
| **Spiromesifen** | 4 | 0 | |
| **Verbindung (I-1-1)/Verbindung (I-1-7)*** + Spiromesifen (1 : 5)** erfindungsgemäß | **0,8 + 4** | **gef.*** **70** | **ber.**** **0** |
| **Sulfoxaflor** | 0,16 | 0 | |
| **Verbindung (I-1-1)/Verbindung (I-1-7)*** + Sulfoxaflor (1 : 1)** erfindungsgemäß | **0,16 + 0,16** | **gef.*** **70** | **ber.**** **0** |
| **Tebufenpyrad** | 0,8 | 0 | |
| **Verbindung (I-1-1)/Verbindung (I-1-7)*** + Tebufenpyrad (1 : 1)** erfindungsgemäß | **0,8 + 0,8** | **gef.*** **70** | **ber.**** **0** |
| **Thiacloprid** | 20 | 80 | |
| **Verbindung (I-1-2)/Verbindung (I-1-8)*** + Thiacloprid (1 : 5)** erfindungsgemäß | **4+20** | **gef.*** **100** | **ber.**** **80** |
| **Thiamethoxam** | 20 | 70 | |
| **Verbindung (I-1-2)/Verbindung (I-1-8)*** + Thiamethoxam (1 : 5)** erfindungsgemäß | **4+20** | **gef.*** **100** | **ber.**** **70** |

(fortgesetzt)

| Wirkstoff | Konzentration in g/ha | Abtötung in % nach 1[d] | |
|---|---|---|---|
| Verbindung (I-1-1)/Verbindung (I-1-7)*** + Thiamethoxam (1 : 5) erfindungsgemäß | 0,8 + 4 | gef.* 90 | ber.** 70 |
| Thiodicarb | 20 | 0 | |
| Verbindung (1-1-2)Verbindung (I-1-8)*** + Thiodicarb (1 : 25) erfindungsgemäß | 0,8 + 20 | gef.* 70 | ber.** 0 |
| Verbindung (I-1-1)/Verbindung (I-1-7)*** + Thiodicarb (1 : 25) erfindungsgemäß | 0,8 + 20 | gef.* 70 | ber.** 0 |
| Transfluthrin | 20 | 70 | |
| Verbindung (I-1-2)/Verbindung (I-1-8)*** + Transfluthrin (1 : 25) erfindungsgemäß | 0,8 + 20 | gef.* 90 | ber.** 70 |
| Triazophos | 100 | 0 | |
| Verbindung (1-1-2)Verbindung (I-1-8)*** + Triazophos (1 : 125) Erfindungsgemäß | 0,8 + 100 | gef.* 70 | ber.** 0 |
| Verbindung (I-1-1)/Verbindung (I-1-7)*** + Triazophos (1 : 125) erfindungsgemäß | 0,8 + 100 | gef.* 70 | ber.** 0 |
| 4-{[(6-CHLORPYRID-3-YL)METHYL](2,2-DIFLUORETHYL)AMINO}FURAN-2(5H)-ON | 20 | 0 | |
| Verbindung (I-1-1)/Verbindung (I-1-7)*** + 4-{[(6-CHLORPYRID-3-YL)METHYL](2,2-DIFLUORETHYL)AMINO} FURAN-2(5H)-ON (125 : 1) erfindungsgemäß | 20+0,16 | gef.* 70 | ber.** 0 |

(fortgesetzt)

| Wirkstoff | Konzentration in g/ha | Abtötung in % nach 1$^{d}$ | |
|---|---|---|---|
| **Pymetrozine** | 100 | 0 | |
| **Verbindung (I-1-1)/Verbindung (I-1-7)*** + Pymetrozine (1 : 25)** erfindungsgemäß | **4 + 100** | **gef.*** **100** | **ber.**** **0** |
| **Cyromazine** | 100 | 0 | |
| **Verbindung (I-1-1)/Verbindung (I-1-7)*** + Cyromazine (1 : 25)** erfindungsgemäß | **4 + 100** | **gef.*** **70** | **ber.**** **0** |

Tabelle A - 2: **Myzus persicae - Test**

| **Wirkstoff** | **Konzentration in g/ha** | **Abtötung in % nach 6[d]** | |
|---|---|---|---|
| **Verbindung (1-1-2)Verbindung (I-1-8)*** ** | 0,16<br>0,032 | 0<br>0 | |
| **3Verbindung (I-1-1)/Verbindung (I-1-7)*** ** | 0,16<br>0,032 | 70<br>0 | |
| **Acetamiprid** | 0,16 | 0 | |
| **Verbindung (I-1-1)/Verbindung (I-1-7)*** + Acetamiprid (1 : 5)**<br>erfindungsgemäß | **0,032 + 0,16** | **gef.***<br>**70** | **ber.****<br>**0** |
| **Aldicarb** | 0,8 | 0 | |
| **Verbindung (1-1-2)Verbindung (I-1-8)*** + Aldicarb (1 : 5)**<br>erfindungsgemäß | **0,16 + 0,8** | **gef.***<br>**80** | **ber.****<br>**0** |
| **Chlorfenapyr** | 0,8<br>0,16 | 0<br>0 | |
| **Verbindung (1-1-2)Verbindung (I-1-8)*** + Chlorfenapyr (1 : 5)**<br>erfindungsgemäß | **0,16 + 0,8** | **gef.***<br>**80** | **ber.****<br>**0** |
| **Verbindung (I-1-1)/Verbindung (I-1-7)*** + Chlorfenapyr (1 : 5)**<br>erfindungsgemäß | **0,032 + 0,16** | **gef.***<br>**70** | **ber.****<br>**0** |
| **Clothianidin** | 0,16 | 0 | |
| **Verbindung (I-1-1)/Verbindung (I-1-7)*** + Clothianidin (1 : 5)**<br>erfindungsgemäß | **0,032 + 0,16** | **gef.***<br>**70** | **ber.****<br>**0** |
| **Cyantraniliprole** | | | |

(fortgesetzt)

| Wirkstoff | Konzentration in g/ha | Abtötung in % nach 6[d] | |
|---|---|---|---|
| | 0,16 | 0 | |
| **Verbindung (I-1-2)/Verbindung (I-1-8)*** + Cyantraniliprole (1 : 5)**<br>Erfindungsgemäß | **0,032 + 0,16** | **gef.***<br>**80** | **ber.****<br>**0** |
| **Verbindung (I-1-1)/Verbindung (I-1-7)*** + Cyantraniliprole (1 : 5)**<br>Erfindungsgemäß | **0,032 + 0,16** | **gef.***<br>**70** | **ber.****<br>**0** |
| **Diafenthiuron** | 20 | 70 | |
| **Verbindung (I-1-2)/Verbindung (I-1-8)*** + Diafenthiuron (1 : 125)**<br>Erfindungsgemäß | **0,16 + 20** | **gef.***<br>**100** | **ber.****<br>**70** |
| **Ethoprophos** | 4 | 0 | |
| **Verbindung (I-1-2)/Verbindung (I-1-8)*** + Ethoprophos (1 : 25)**<br>Erfindungsgemäß | **0,16+4** | **gef.***<br>**80** | **ber.****<br>**0** |
| **Fenamiphos** | | | |
| | 0,8 | 0 | |
| **Verbindung (I-1-1)/Verbindung (I-1-7)*** + Fenamiphos (1 : 25)**<br>**Erfindungsgemäß** | **0,032 + 0,8** | **gef.***<br>**70** | **ber.****<br>**0** |
| **11-(4-CHLOR-2,6-DIMETHYLPHENYL)-12-HYDROXY-1,4-DIOXA-9-AZADISPIRO[4.2.4.2]TETRADEC -11-EN-10-ON** | 0,8 | 0 | |
| **Verbindung (I-1-1)/Verbindung (I-1-7)*** + 11-(4-CHLOR-2,6-DIMETHYLPHENYL)-12-HYDROXY-1,4-DIOXA-9-AZADISPIRO[4.2.4.2]TETRADEC -11-EN-10-ON (1 : 25)**<br>Erfindungsgemäß | **0,032 + 0,8** | **gef.***<br>**70** | **ber.****<br>**0** |
| **Fipronil** | 0,16 | 0 | |

(fortgesetzt)

| Wirkstoff | Konzentration in g/ha | Abtötung in % nach 6[d] | |
|---|---|---|---|
| **Verbindung (I-1-2)/Verbindung (I-1-8)*** + Fipronil(1 : 1)**<br>Erfindungsgemäß | **0,16 + 0,16** | **gef.*<br>80** | **ber.**<br>0** |
| **Flufenoxuron** | 0,8 | 0 | |
| **Verbindung (I-1-1)/Verbindung (I-1-7)*** + Flufenoxuron(1 : 25)**<br>Erfindungsgemäß | **0,032 + 0,8** | **gef.*<br>70** | **ber.**<br>0** |
| **Methoxyfenozide** | 4 | 0 | |
| **Verbindung (I-1-2)/Verbindung (I-1-8)*** + Methoxyfenozide (1 : 25)**<br>Erfindungsgemäß | **0,16+4** | **gef.*<br>100** | **ber.**<br>0** |
| **Milbemectin** | 0,032 | 0 | |
| **Verbindung (I-1-1)/Verbindung (I-1-7)*** + Milbemectin (1 : 1)**<br>Erfindungsgemäß | **0,032 + 0,032** | **gef.*<br>70** | **ber.**<br>0** |
| **Tebufenozide** | 4 | 0 | |
| **Verbindung (I-1-2)/Verbindung (I-1-8)*** + Tebufenozide (1 : 25)**<br>Erfindungsgemäß | **0,16+4** | **gef.*<br>100** | **ber.**<br>0** |
| **Tebufenpyrad** | 0,16 | 0 | |
| **Verbindung (I-1-2)/Verbindung (I-1-8)*** + Tebufenpyrad (1 : 1)**<br>Erfindungsgemäß | **0,16 + 0,16** | **gef.*<br>80** | **ber.**<br>0** |
| **Triflumuron** | | | |

(fortgesetzt)

| Wirkstoff | Konzentration in g/ha | Abtötung in % nach 6[d] | |
|---|---|---|---|
| | 20<br>4 | 0<br>0 | |
| **Verbindung (I-1-2)/Verbindung (I-1-8)*** + Triflumuron(1 : 125)** | **0,16 + 20** | **gef.***<br>**90** | **ber.****<br>**0** |
| Erfindungsgemäß | | | |
| **Verbindung (I-1-1)/Verbindung (I-1-7)*** + Triflumuron(1 : 125)**<br>Erfindungsgemäß | **0,032 + 4** | **gef.***<br>**70** | **ber.****<br>**0** |
| **Spinetoram** | 0,8 | 0 | |
| **Verbindung (I-1-1)/Verbindung (I-1-7)*** + Spinetoram (1 : 5)**<br>Erfindungsgemäß | **0,16 + 0,8** | **gef.***<br>**90** | **ber.****<br>**70** |
| **Thiacloprid** | 0,8 | 80 | |
| **Verbindung (I-1-1)/Verbindung (I-1-7)*** + Thiacloprid (1 : 25)**<br>erfindungsgemäß | **0,032 + 0,8** | **gef.***<br>**100** | **ber.****<br>**80** |
| **2-{6-[2-(5-FLUORPYRIDIN-3-YL)-1,3-THIAZOL-5-YL]PYRIDIN-2-YL}PYRIMIDIN** | 0,8 | 70 | |
| **Verbindung (I-1-1)/Verbindung (I-1-7)*** + 2-{6-[2-(5-FLUORPYRIDIN-3-YL)-1,3-THIAZOL-5-YL]PYRIDIN-2-YL} PYRIMIDIN (1 : 25)**<br>erfindungsgemäß | **0,032 + 0,8** | **gef.***<br>**100** | **ber.****<br>**70** |

* gef. = gefundene Wirkung
** ber. = nach der Colby-Formel berechnete Wirkung
*** In den getesteten Mischungen aus Verbindung (I-1-1)/Verbindung (1-1-7) bzw. Verbindung (1-1-2)/Verbindung (1-1-8) lagen die Verbindungen (1-1-1) bzw. (1-1-2) jeweils zu ca 85 % bzw. ca 84 % vor, die Verbindungen (1-1-7) bzw. (1-1-8) zu jeweils ca 15 %.

Beispiel **B**

**Phaedon cochleariae - Larven -Test**

Lösungsmittel: 78 Gewichtsteile Aceton
1,5 Gewichtsteile Dimethylformamid

Emulgator: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (*Brassica oleracea*) werden durch Spritzen mit der Wirkstoffzubereitung in der gewünschten Konzentration behandelt und mit Larven des Meerrettichblattkäfers (*Phaedon cochleariae)* besetzt, solange die Blätter noch feucht sind.

Nach der gewünschten Zeit wird die Abtötung in % bestimmt. Dabei bedeutet 100 %, dass alle Käferlarven abgetötet wurden; 0 % bedeutet, dass keine Käferlarven abgetötet wurden. Die ermittelten Abtötungswerte verrechnet man nach der Colby-Formel.

Bei diesem Test zeigen die folgenden Wirkstoffkombinationen gemäß vorliegender Anmeldung eine synergistisch verstärkte Wirksamkeit im Vergleich zu den einzeln angewendeten Wirkstoffen:

**Tabelle B - 1: Phaedon cochleariae Larven - Test**

| Wirkstoff | Konzentration in g/ha | Abtötung in % nach 2$^d$ | |
|---|---|---|---|
| **Verbindung (I-1-2)/Verbindung (I-1-8)*** ** | 0,16<br>0,032 | 50<br>0 | |
| **Verbindung (I-1-1)/Verbindung (I-1-7)*** ** | 0,16 | 67 | |
| **Aldicarb** | 0,8 | 0 | |
| **Verbindung (I-1-1)/Verbindung (I-1-7)*** ** + **Aldicarb (1 : 5)**<br>erfindungsgemäß | **0,16 + 0,8** | **gef.***<br>**100** | **ber.****<br>**67** |
| β-**Cyfluthrin** | 0,032 | 0 | |
| **Verbindung (I-1-2)/Verbindung (I-1-8)*** ** + β-**Cyfluthrin** (5 : 1)<br>erfindungsgemäß | **0,16 + 0,032** | **gef.***<br>**67** | **ber.****<br>**50** |
| **Cyantraniliprole** | 0,16 | 0 | |
| **Verbindung (I-1-2)/Verbindung (I-1-8)*** + Cyantraniliprole (1 : 5)**<br>erfindungsgemäß | **0,032 + 0,16** | **gef.***<br>**33** | **ber.****<br>**0** |
| **Dinotefuran** | 20 | 0 | |
| **Verbindung (I-1-2)/Verbindung (I-1-8)*** + Dinotefuran (1 : 125)** erfindungsgemäß | **0,16 + 20** | **gef.***83 | **ber.**** 50 |
| **Verbindung (I-1-1)/Verbindung (I-1-7)*** ** + **Dinotefuran (1 : 125)**<br>erfindungsgemäß | **0,16 + 20** | **gef.***<br>**100** | **ber.****<br>**67** |

(fortgesetzt)

| Wirkstoff | Konzentration in g/ha | Abtötung in % nach 2[d] | |
|---|---|---|---|
| **Fipronil** | 0,16 | 67 | |
| **Verbindung (I-1-2)/Verbindung (I-1-8)*** + Fipronil (1 : 1)**<br>erfindungsgemäß | **0,16 + 0,16** | **gef.***<br>**100** | **ber.****<br>**83,5** |
| **Verbindung (I-1-1)/Verbindung (I-1-7)*** + Fipronil (1 : 1)**<br>erfindungsgemäß | **0,16 + 0,16** | **gef.***<br>**100** | **ber.****<br>**83,5** |
| **Profenophos** | 4 | 0 | |
| **Verbindung (I-1-2)/Verbindung (I-1-8)*** + Profenophos (1 : 25)**<br>erfindungsgemäß | **0,16 + 4** | **gef.***<br>**83** | **ber.****<br>**50** |
| **Tebufenozide** | 4 | 0 | |
| **Verbindung (I-1-2)/Verbindung (I-1-8)*** + Tebufenozide (1 : 1)**<br>erfindungsgemäß | **0,16 + 4** | **gef.***<br>**83** | **ber.****<br>**50** |

* gef. = gefundene Wirkung
** ber. = nach der Colby-Formel berechnete Wirkung
*** In den getesteten Mischungen aus Verbindung (I-1-1)/Verbindung (I-1-7) bzw. Verbindung (I-1-2)/Verbindung (I-1-8) lagen die Verbindungen (I-1-1) bzw. (I-1-2) jeweils zu ca 85 % bzw. ca 84 % vor, die Verbindungen (I-1-7) bzw. (I-1-8) zu jeweils ca 15 %.

Tabelle B - 2: **Phaedon cochleariae Larven - Test**

| Wirkstoff | Konzentration in g/ha | Abtötung in % nach 6[d] | |
|---|---|---|---|
| **Verbindung (I-1-2)/Verbindung (I-1-8)*** ** | 0,032 | 0 | |
| **Verbindung (I-1-1)Nerbindung (I-1-7)*** ** | 0,16<br>0,032 | 83<br>0 | |
| **Abamectin** | 0,032 | 0 | |
| **Verbindung (I-1-1)/Verbindung (I-1-7)*** + Abamectin (5 : 1)**<br>**Erfindungsgemäß** | **0,16 + 0,032** | **gef.***<br>**100** | **ber.****<br>**83** |
| **Acephate** | 4 | 0 | |
| **Verbindung (I-1-1)/Verbindung (I-1-7)*** + Acephate (1 : 25)**<br>Erfindungsgemäß | **0,16 + 4** | **gef.***<br>**100** | **ber.****<br>**83** |
| **Cyantraniliprole** | 0,16 | 0 | |
| **Verbindung (I-1-2)/Verbindung (I-1-8)*** + Cyantraniliprole (1 : 5)** erfindungsgemäß | **0,032 + 0,16** | **gef.***<br>**50** | **ber.****<br>**0** |
| **Verbindung (I-1-1)/Verbindung (I-1-7)*** + Cyantraniliprole (1 : 5)**<br>Erfindungsgemäß | **0,032 + 0,16** | **gef.***<br>**100** | **ber.****<br>**0** |
| **Cypermethrin** | 0,16 | 0 | |
| **Verbindung (I-1-2)/Verbindung (I-1-8)*** + Cypermethrin (1 : 5)**<br>erfindungsgemäß | **0,032 + 0,16** | **gef.***<br>**33** | **ber.****<br>**0** |
| **Emamectin - benzoate** | 0,032 | 0 | |
| **Verbindung (I-1-1)/Verbindung (I-1-7)*** + Emamectin-benzoate (5 : 1)**<br>Erfindungsgemäß | **0,16 + 0,032** | **gef.***<br>**100** | **ber.****<br>**83** |

(fortgesetzt)

| Wirkstoff | Konzentration in g/ha | Abtötung in % nach 6$^{d}$ | |
|---|---|---|---|
| **Ethoprophos** | 0,8 | 0 | |
| **Verbindung (I-1-2)/Verbindung (I-1-8)*** + Ethoprophos (1 : 25)**<br>erfindungsgemäß | **0,032 + 0,8** | **gef.***<br>**33** | **ber.****<br>**0** |
| **Flubendiamid** | 0,8 | 0 | |
| **Verbindung (I-1-1)/Verbindung (I-1-7)*** + Flubendiamid (1 : 5)**<br>Erfindungsgemäß | **0,16 + 0,8** | **gef.***<br>**100** | **ber.****<br>**83** |
| **Novaluron** | 0,8 | 0 | |
| **Verbindung (I-1-1)/Verbindung (I-1-7)*** + Novaluron (1 : 5)**<br>Erfindungsgemäß | **0,16 + 0,8** | **gef.***<br>**100** | **ber.****<br>**83** |
| **Profenophos** | 4 | 0 | |
| **Verbindung (I-1-1)/Verbindung (I-1-7)*** + Profenophos (1 : 25)**<br>Erfindungsgemäß | **0,16 + 4** | **gef.***<br>**100** | **ber.****<br>**83** |
| **Spirodiclofen** | 4 | 0 | |
| **Verbindung (I-1-1)/Verbindung (I-1-7)*** + Spirodiclofen (1 : 25)**<br>Erfindungsgemäß | **0,16 + 4** | **gef.***<br>**100** | **ber.****<br>**83** |
| **Tebufenpyrad** | 0,16 | 0 | |
| **Verbindung (I-1-1)/Verbindung (I-1-7)*** + Tebufenpyrad (1 : 1)** | **0,16 + 0,16** | **gef.***<br>**100** | **ber.****<br>**83** |

(fortgesetzt)

| **Wirkstoff** | **Konzentration in g/ha** | **Abtötung in % nach 6$^d$** |
|---|---|---|
| Erfindungsgemäß | | |
| **Triflumuron** | 4 | 0 |
| **Verbindung (I-1-2)/Verbindung (I-1-8)*** + Triflumuron (1 : 125)**<br>Erfindungsgemäß | **0,032 + 4** | **gef.*** **ber.****<br>**50 0** |
| **Spinetoram** | 0,16 | 0 |
| **Verbindung (I-1-1)/Verbindung (I-1-7)*** + Spinetoram (1 : 5)** | **0,032 + 0,16** | **gef.* 33** **ber.** 0** |
| Erfindungsgemäß | | |
| **Pyridalyl** | 4 | 0 |
| **Verbindung (I-1-1)/Verbindung (I-1-7)*** + Pyridalyl (1 : 25)**<br>Erfindungsgemäß | **0,16 + 4** | **gef.*** **ber.****<br>**100** **83** |
| **Methoxyfenozide** | 0,8 | 0 |
| **Verbindung (I-1-1)/Verbindung (1-1-7)*** + Methoxyfenozide (1 : 5)**<br>Erfindungsgemäß | **0,16 + 0,8** | **gef.*** **ber.****<br>**100** **83** |
| **2-{6-[2-(5-FLUORPYRIDIN-3-YL)-1,3-THIAZOL-5-YL]PYRIDIN-2-YL}PYRIMIDIN** | 0,16 | 0 |
| **Verbindung (I-1-1)/Verbindung (I-1-7)*** + 2-{6-[2-(5-FLUORPYRIDIN-3-YL)-1,3-THIAZOL-5-YL]PYRIDIN-2-YL} PYRIMIDIN (1 : 25)**<br>Erfindungsgemäß | **0,16 + 4** | **gef.*** **ber.****<br>**100** **83** |
| **Cyromazine** | 4 | 0 |
| **Verbindung (I-1-1)/Verbindung (I-1-7)*** + Cyromazine (1 : 25)**<br>Erfindungsgemäß | **0,16 + 4** | **gef.*** **ber.****<br>**100** **83** |
| **Cyflumetofen** | | |

(fortgesetzt)

| Wirkstoff | Konzentration in g/ha | Abtötung in % nach $6^d$ | |
|---|---|---|---|
| | 4 | 0 | |
| **Verbindung (I-1-1)/Verbindung (I-1-7)*** + Cyflumetofen (1 : 25)** Erfindungsgemäß | **0,16 + 4** | **gef.*** **100** | **ber.**** **83** |

* gef. = gefundene Wirkung
** ber. = nach der Colby-Formel berechnete Wirkung
*** In den getesteten Mischungen aus Verbindung (I-1-1)/Verbindung (I-1-7) bzw. Verbindung (I-1-2)/Verbindung (I-1-8) lagen die Verbindungen (I-1-1) bzw. (I-1-2) jeweils zu ca 85 % bzw. ca 84 % vor, die Verbindungen (I-1-7) bzw. (I-1-8) zu jeweils ca 15 %.

Beispiel C

**Spodoptera frugiperda - Larven -Test**

Lösungsmittel: 78 Gewichtsteile Aceton
1,5 Gewichtsteile Dimethylformamid

Emulgator: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (*Brassica oleracea*) werden durch Spritzen mit der Wirkstoffzubereitung in der gewünschten Konzentration behandelt und mit Larven des Heerwurms (*Spodoptera frugiperda*) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden. Die ermittelten Abtötungswerte verrechnet man nach der Colby-Formel.

Bei diesem Test zeigen die folgenden Wirkstoffkombinationen gemäß vorliegender Anmeldung eine synergistisch verstärkte Wirksamkeit im Vergleich zu den einzeln angewendeten Wirkstoffen:

**Tabelle C - 1: Spodoptera frugiperda Larven - Test**

| Wirkstoff | Konzentration in g/ha | Abtötung in % nach 2$^{d}$ | |
|---|---|---|---|
| **Verbindung (I-1-2)/Verbindung (I-1-8)*** ** | 0,16<br>0,032 | 33<br>17 | |
| **Fenpyroximate** | 0,8 | 0 | |
| **Verbindung (I-1-2)/Verbindung (I-1-8)*** + Fenpyroximate (1 : 5)**<br>erfindungsgemäß | **0,16 + 0,8** | **gef.***<br>**83** | **ber.****<br>**33** |
| **Gamma-Cyhalothrin** | 0,032 | 17 | |
| **Verbindung (I-1-2)/Verbindung (I-1-8)*** + Fenpyroximate (5 : 1)**<br>erfindungsgemäß | **0,16 + 0,032** | **gef.***<br>**83** | **ber.****<br>**44,39** |
| **Indoxacarb** | 0,8 | 50 | |
| **Verbindung (I-1-2)/Verbindung (I-1-8)*** + Indoxacarb (1 : 25)**<br>Erfindungsgemäß | **0,032 + 0,8** | **gef.***<br>**83** | **ber.****<br>**58,5** |
| **Triazophos** | 4 | 0 | |
| **Verbindung (I-1-2)/Verbindung (I-1-8)*** + Triazophos (1 : 125)**<br>Erfindungsgemäß | **0,032 + 4** | **gef.***<br>**67** | **ber.****<br>**17** |

* gef. = gefundene Wirkung
** ber. = nach der Colby-Formel berechnete Wirkung
*** In den getesteten Mischungen aus Verbindung (I-1-1)/Verbindung (I-1-7) bzw. Verbindung (I-1-2)/Verbindung (I-1-8) lagen die Verbindungen (I-1-1) bzw. (I-1-2) jeweils zu ca 85 % bzw. ca 84 % vor, die Verbindungen (I-1-7) bzw. (I-1-8) zu jeweils ca 15 %.

Tabelle C - 2:

| Wirkstoff | Konzentration in g/ha | Abtötung in % nach 6d | |
|---|---|---|---|
| **Verbindung (I-1-1)/Verbindung (I-1-7)*** ** | 0,16<br>0,032 | 33<br>0 | |
| **Carbaryl** | 4 | 0 | |
| **Verbindung (I-1-1)/Verbindung (I-1-7)*** + Carbaryl (1 : 125)**<br>Erfindungsgemäß | **0,032 + 4** | **gef.***<br>**50** | **ber.****<br>**0** |
| **Fluensulfone** | 2000 | 0 | |
| **Verbindung (I-1-1)/Verbindung (I-1-7)*** + Fluensulfone (1 : 12500)**<br>Erfindungsgemäß | **0,16 + 2000** | **gef.***<br>**67** | **ber.****<br>**33** |
| **Flufenoxuron** | 0,8 | 0 | |
| **Verbindung (I-1-1)/Verbindung (I-1-7)*** + Flufenoxuron (1 : 25)**<br>Erfindungsgemäß | **0,032 + 0,8** | **gef.***<br>**100** | **ber.****<br>**0** |
| **Imicyafos** | 45 | 0 | |
| **Verbindung (I-1-1)/Verbindung (I-1-7)*** + Imicyafos (1 : 281,25)**<br>Erfindungsgemäß | **0,16 + 45** | **gef.***<br>**83** | **ber.****<br>**33** |
| **L-Cyhalothrin** | 0,032 | 0 | |
| **Verbindung (I-1-1)/Verbindung (I-1-7)*** + L-Cyhalothrin (1 : 1)**<br>Erfindungsgemäß | **0,032 + 0,032** | **gef.***<br>**83** | **ber.****<br>**0** |
| **Lufenuron** | 0,8 | 17 | |
| **Verbindung (I-1-1)/Verbindung (I-1-7)*** + Lufenuron (1 : 25)**<br>Erfindungsgemäß | **0,032 + 0,8** | **gef.***<br>**67** | **ber.****<br>**17** |
| **Novaluron** | 0,8 | 67 | |
| **Verbindung (I-1-1)/Verbindung (I-1-7)*** + Novaluron (1 : 25)**<br>Erfindungsgemäß | **0,032 + 0,8** | **gef.***<br>**83** | **ber.****<br>**67** |
| **Profenophos** | 4 | 17 | |
| **Verbindung (I-1-1)/Verbindung (I-1-7)*** + Profenophos (1 : 125)**<br>Erfindungsgemäß | **0,032 + 4** | **gef.***<br>**67** | **ber.****<br>**17** |
| **Chloranthraniliprole** | 0,032 | 50 | |

(fortgesetzt)

| Wirkstoff | Konzentration in g/ha | Abtötung in % nach 6[d] | |
|---|---|---|---|
| **Verbindung (I-1-1)/Verbindung (I-1-7)*** + Chloranthraniliprole (1 : 1)** Erfindungsgemäß | **0,032 + 0,032** | **gef.* 100** | **ber.** 50** |
| **Spinosad** | 0,16 | 0 | |
| **Verbindung (I-1-1)/Verbindung (I-1-7)*** + Spinosad (1 : 5)** Erfindungsgemäß | **0,032 + 0,16** | **gef.* 33** | **ber.** 0** |
| **Tebufenozide** | 0,16 | 0 | |
| **Verbindung (I-1-1)/Verbindung (I-1-7)*** + Tebufenozide (1 : 5)** erfindungsgemäß | **0,032 + 0,16** | **gef.* 33** | **ber.** 0** |
| **Pyridalyl** | 4 | 0 | |
| **Verbindung (I-1-1)/Verbindung (I-1-7)*** + Pyridalyl (1 : 25)** erfindungsgemäß | **0,16 + 4** | **gef.* 50** | **ber.** 33** |
| **Methoxyfenozide** | 0,8 | 0 | |
| **Verbindung (I-1-1)/Verbindung (I-1-7)*** + Methoxyfenozide (1 : 5)** erfindungsgemäß | **0,16 + 0,8** | **gef.* 67** | **ber.** 33** |
| **Cyromazine** | 4 | 0 | |
| **Verbindung (I-1-1)/Verbindung (I-1-7)*** + Cyromazine (1 : 25)** erfindungsgemäß | **0,16 + 4** | **gef.* 50** | **ber.** 33** |
| **Cyflumetofen** | 4 | 17 | |
| **Verbindung (I-1-1)/Verbindung (I-1-7)*** + Cyflumetofen (1 : 25)** erfindungsgemäß | **0,16 + 4** | **gef.* 50** | **ber.** 33** |

* gef. = gefundene Wirkung
** ber. = nach der Colby-Formel berechnete Wirkung
*** In den getesteten Mischungen aus Verbindung (I-1-1)/Verbindung (I-1-7) bzw. Verbindung (I-1-2)/Verbindung (I-1-8) lagen die Verbindungen (I-1-1) bzw. (I-1-2) jeweils zu ca 85 % bzw. ca 84 % vor, die Verbindungen (I-1-7) bzw. (I-1-8) zu jeweils ca 15 %.

Beispiel D

**Tetranychus-Test** (OP-resistent/Spritzbehandlung)

Lösungsmittel: 78 Gewichtsteile Aceton

1,5 Gewichtsteile Dimethylformamid

Emulgator: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben (Phaseolus vulgaris), die von allen Stadien der Gemeinen Spinnmilbe (Tetranychus urticae) befallen sind, werden mit einer Wirkstoffzubereitung in der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigte die folgende Wirkstoffkombination gemäß vorliegender Anmeldung eine synergistisch verstärkte Wirksamkeit im Vergleich zu den einzeln angewendeten Wirkstoffen:

Tabelle D - 1: **Tetranychus urticae - Test**

| **Wirkstoff** | **Konzentration in g/ha** | **Abtötung in % nach 2^d^** | |
|---|---|---|---|
| **Verbindung (I-1-2)/Verbindung (I-1-8)***** | 4<br>0,8<br>0,16<br>0,032 | 0<br>0<br>0<br>0 | |
| **Verbindung (I-1-1)/Verbindung (I-1-7)***** | 4<br>0,8<br>0,16<br>0,032 | 0<br>0<br>0<br>0 | |
| **Acrinathrin** | 4<br>0,8 | 20<br>0 | |
| **Verbindung (I-1-2)/Verbindung (I-1-8)*** + Acrinathrin (1 : 25)**<br>erfindungsgemäß | **0,16 + 4** | **gef.***<br>**70** | **ber.****<br>**20** |
| **Alpha-Cypermethrin** | 4 | 0 | |
| **Verbindung (I-1-2)/Verbindung (I-1-8)*** + Alpha-Cypermethrin (1 : 1)**<br>Erfindungsgemäß | **4+4** | **gef.***<br>**80** | **ber.****<br>**0** |
| **Verbindung (I-1-1)/Verbindung (I-1-7)*** + Alpha-Cypermethrin (1 : 1)**<br>Erfindungsgemäß | **4+4** | **gef.***<br>**70** | **ber.****<br>**0** |
| **Bifenthrin** | | | |
| | 0,16 | 0 | |
| **Verbindung (1-1-2)/Verbindung (I-1-8)*** + Bifenthrin (1 : 1)**<br>Erfindungsgemäß | **0,16 + 0,16** | **gef.***<br>**70** | **ber.****<br>**0** |
| **Verbindung (I-1-1)/Verbindung (I-1-7)*** + Bifenthrin (1 : 1)** | **0,16 + 0,16** | **gef.***<br>**40** | **ber.****<br>**0** |

(fortgesetzt)

| Wirkstoff | Konzentration in g/ha | Abtötung in % nach 2d |
|---|---|---|
| Erfindungsgemäß | | |
| **Carbaryl** | 500 | 10 |
| **Verbindung (1-1-2)/Verbindung (I-1-8)*** + Carbaryl (1 : 125)**<br>Erfindungsgemäß | **4 + 500** | **gef.* 50** **ber.** 10** |
| **Chlorfenapyr** | 4 | 10 |
| **Verbindung (1-1-2)/Verbindung (I-1-8)*** + Chlorfenapyr (1 : 5)**<br>Erfindungsgemäß | **0,8 + 4** | **gef.* 70** **ber.** 10** |
| **Verbindung** (I-1-1)/**Verbindung (I-1-7)***** + **Chlorfenapyr (1 : 5)**<br>Erfindungsgemäß | **0,8 + 4** | **gef.* 80** **ber.** 10** |
| **Diafenthiuron** | 100 | 0 |
| **Verbindung (1-1-2)/Verbindung (I-1-8)*** + Diafenthiuron (1 : 125)**<br>Erfindungsgemäß | **0,8 + 100** | **gef.* 70** **ber.** 0** |
| **Emamectin-Benzoate** | 0,032 | 0 |
| **Verbindung (1-1-2)/Verbindung (I-1-8)*** + Emamectin-Benzoate (5 : 1)**<br>Erfindungsgemäß | **0,16 + 0,032** | **gef.* 30** **ber.** 0** |
| **Fenamiphos** | 20 | 0 |
| **Verbindung (1-1-2)/Verbindung (I-1-8)*** + Fenamiphos (1 : 25)** | **0,8 + 20** | **gef.* 20** **ber.** 0** |

(fortgesetzt)

| Wirkstoff | Konzentration in g/ha | Abtötung in % nach 2d |
|---|---|---|
| Erfindungsgemäß | | |
| **Fenpyroximate** | 0,8 | 0 |
| **Verbindung (1-1-2)/Verbindung (I-1-8)*** + Fenpyroximate (1 : 25)**<br>Erfindungsgemäß | **0,032 + 0,8** | **gef.* 50** **ber.** 0** |
| **Verbindung (I-1-1)/Verbindung (I-1-7)*** + Fenpyroximate (1 : 25)**<br>Erfindungsgemäß | **0,032 + 0,8** | **gef.* 80** **ber.** 0** |
| **Flubendiamide** | 20 | 0 |
| **Verbindung (I-1-1)/Verbindung (I-1-7)*** + Flubendiamide (1 : 5)**<br>Erfindungsgemäß | **4+20** | **gef.* 30** **ber.** 0** |
| **Fluensulfone** | 2000 | 0 |
| **Verbindung (I-1-1)/Verbindung (I-1-7)*** + Fluensulfone (1 : 500)**<br>Erfindungsgemäß | **4 + 2000** | **gef.* 30** **ber.** 0** |
| **Gamma - Cyhalothrin** | 0,8 | 0 |
| **Verbindung (I-1-1)/Verbindung (I-1-7)*** + Gamma-Cyhalothrin (1 : 1)**<br>Erfindungsgemäß | **0,8 + 0,8** | **gef.* 30** **ber.** 0** |
| **Lufenuron** | 100 | 0 |
| **Verbindung (I-1-1)/Verbindung (I-1-7)*** + Lufenuron (1 : 25)** | **4 + 100** | **gef.* 20** **ber.** 0** |

(fortgesetzt)

| Wirkstoff | Konzentration in g/ha | Abtötung in % nach 2[d] |
|---|---|---|
| Erfindungsgemäß | | |
| **Milbemectin** | 0,032 | 50 |
| **Verbindung (I-1-1)/Verbindung (I-1-7)*** + Milbemectin (1 : 1)**<br>Erfindungsgemäß | **0,032 + 0,032** | **gef.*** **ber.****<br>**80** **0** |
| **Spinosad** | 20 | 20 |
| **Verbindung (1-1-2)/Verbindung (I-1-8)***** + **Spinosad (1 : 5)**<br>Erfindungsgemäß | **4+20** | **gef.*** **ber.****<br>**60** **20** |
| **Spirodiclofen** | 100 | 0 |
| **Verbindung (1-1-2)/Verbindung (I-1-8)***** + **Spirodiclofen (1 : 25)**<br>Erfindungsgemäß | **4 + 100** | **gef.*** **ber.****<br>**70** **0** |
| **Verbindung (I-1-1)/Verbindung (I-1-7)*** + Spirodiclofen (1 : 25)**<br>Erfindungsgemäß | **4 + 100** | **gef.*** **ber.****<br>**80** **0** |
| **Spirotetramat** | 4 | 0 |
| **Verbindung (1-1-2)/Verbindung (I-1-8)*** + Spirotetramat (1 : 5)**<br>Erfindungsgemäß | **0,8 + 4** | **gef.*** **ber.****<br>**20** **0** |
| **Tebufenpyrad** | 0,16 | 0 |
| **Verbindung (I-1-1)/Verbindung (I-1-7)*** + Tebufenpyrad (1 : 5)** | **0,032 + 0,16** | **gef.*** **ber.****<br>**40** **0** |

(fortgesetzt)

| Wirkstoff | Konzentration in g/ha | Abtötung in % nach 2d |
|---|---|---|
| Erfindungsgemäß | | |
| **Thiodicarb** | 100 | 0 |
| **Verbindung (1-1-2)/Verbindung (I-1-8)*** + Thiodicarb (1 : 25)**<br>Erfindungsgemäß | 4 + 100 | gef.* ber.**<br>50 0 |
| **Verbindung (I-1-1)/Verbindung (I-1-7)*** + Thiodicarb (1 : 25)**<br>Erfindungsgemäß | 4 + 100 | gef.* ber.**<br>70 0 |
| **4-{[(6-CHLORPYRID-3-YL)METHYL](2,2-DIFLUORETHYL)AMINO}FURAN -2(5H)-ON** | 20 | 0 |
| **Verbindung (I-1-1)/Verbindung (I-1-7)*** + 4-{[(6-CHLORPYRID-3-YL)METHYL](2,2-DIFLUORETHYL)AMINO}FURAN -2(5H)-ON (1 : 5)** | 4+20 | gef.* ber.**<br>20 0 |
| Erfindungsgemäß | | |

* gef. = gefundene Wirkung
** ber. = nach der Colby-Formel berechnete Wirkung
*** In den getesteten Mischungen aus Verbindung (I-1-1)/Verbindung (1-1-7) bzw. Verbindung (1-1-2)/Verbindung (1-1-8) lagen die Verbindungen (1-1-1) bzw. (1-1-2) jeweils zu ca 85 % bzw. ca 84 % vor, die Verbindungen (1-1-7) bzw. (1-1-8) zu jeweils ca 15 %.

Tabelle D - 2:

| Wirkstoff | Konzentration in g/ha | Abtötung in % nach 6[d] | |
|---|---|---|---|
| **Verbindung (1-1-2)/Verbindung (I-1-8)*** ** | 4<br>0,8<br>0,16<br>0,032 | 0<br>0<br>0<br>0 | |
| **Verbindung** (I-1-1)/**Verbindung (I-1-7)*** ** | 4<br>0,8<br>0,16<br>0,032 | 10<br>10<br>0<br>0 | |
| **Acrinathrin** | 0,8 | 40 | |
| **Verbindung (1-1-2)/Verbindung (I-1-8)***+ Acrinathrin (1 : 25)**<br>Erfindungsgemäß | **0,032 + 0,8** | **gef.***<br>**70** | **ber.****<br>**40** |
| **Verbindung** (I-1-1)/**Verbindung (I-1-7)***+ Acrinathrin (1 : 25)**<br>Erfindungsgemäß | **0,032 + 0,8** | **gef.***<br>**80** | **ber.****<br>**40** |
| **Abamectin** | 0,032<br>0,0064 | 80<br>0 | |
| **Verbindung (1-1-2)/Verbindung (I-1-8)***+ Abamectin (5 : 1)**<br>Erfindungsgemäß | **0,16 + 0,032** | **gef.***<br>**100** | **ber.****<br>**80** |
| **Verbindung** (I-1-1)/**Verbindung (I-1-7)***+ Abamectin** (5 : **1)**<br>Erfindungsgemäß | **0,032 + 0,0064** | **gef.***<br>**70** | **ber.****<br>**0** |
| **Cadusaphos** | 20 | 0 | |
| **Verbindung (1-1-2)/Verbindung (I-1-8)***+ Cadusaphos (1 : 25)**<br>Erfindungsgemäß | **0,8** + **20** | **gef.***<br>**40** | **ber.****<br>**0** |
| **Carbaryl** | 500 | 20 | |
| **Verbindung (1-1-2)/Verbindung** (I-1-8)***+ **Carbaryl (1 : 125)**<br>Erfindungsgemäß | **4 + 500** | **gef.***<br>**70** | **ber.****<br>**20** |
| **Chlorpyrifos** | 100 | 0 | |
| **Verbindung (1-1-2)/Verbindung (I-1-8)***+ Chlorpyrifos (1 : 25)**<br>Erfindungsgemäß | **4+100** | **gef.***<br>**30** | **ber.****<br>**0** |
| **Verbindung** (I-1-1)/**Verbindung (I-1-7)***+ Chlorpyrifos (1** : 25)<br>Erfindungsgemäß | **4+100** | **gef.***<br>**70** | **ber.****<br>**10** |
| **Diafenthiuron** | 20 | 10 | |
| **Verbindung (1-1-2)/Verbindung (I-** | | **gef.*** | **ber.**** |

(fortgesetzt)

| Wirkstoff | Konzentration in g/ha | Abtötung in % nach 6d | |
|---|---|---|---|
| **1-8)***+ Diafenthiuron (1 : 125)** Erfindungsgemäß | **0,16 + 20** | **30** | **10** |
| **L-Cyhalothrin** | 4 | 20 | |
| **Verbindung** (I-1-1)/**Verbindung (I-1-7)***+ L-Cyhalothrin** (1 : 1) Erfindungsgemäß | **4+4** | **gef.*** **70** | **ber.**** **28** |
| **Spinetoram** | 4 | 20 | |
| **Verbindung (1-1-2)/Verbindung (I-1-8)***+ Spinetoram (1 : 1)** Erfindungsgemäß | **4+4** | **gef.*** **80** | **ber.**** **20** |
| **Spiromesifen** | 20 4 | 80 70 | |
| **Verbindung (1-1-2)/Verbindung (I-1-8)***+ Spiromesifen (1 : 5)** Erfindungsgemäß | **4+20** | **gef.*** **100** | **ber.**** **80** |
| **Verbindung** (I-1-1)/**Verbindung (I-1-7)***+ Spiromesifen(1** : **5)** Erfindungsgemäß | **0,8 + 4** | **gef.*** **90** | **ber.**** **73** |
| **Tebufenpyrad** | 4 | 40 | |
| **Verbindung (1-1-2)/Verbindung (I-1-8)***+ Tebufenpyrad (1 : 1)** Erfindungsgemäß | **4+4** | **gef.*** **90** | **ber.**** **40** |
| **Verbindung** (I-1-1)/**Verbindung (I-1-7)***+ Tebufenpyrad (1 : 1)** Erfindungsgemäß | **4+4** | **gef.*** **80** | **ber.**** **46** |
| **Tefluthrin** | 20 | 0 | |
| **Verbindung (1-1-2)/Verbindung (I-1-8)***+ Tefluthrin (1 : 5)** Erfindungsgemäß | **4+20** | **gef.*** **40** | **ber.**** **0** |

* gef. = gefundene Wirkung
** ber. = nach der Colby-Formel berechnete Wirkung
*** In den getesteten Mischungen aus Verbindung (I-1-1)/Verbindung (1-1-7) bzw. Verbindung (1-1-2)/Verbindung (1-1-8) lagen die Verbindungen (1-1-1) bzw. (1-1-2) jeweils zu ca 85 % bzw. ca 84 % vor, die Verbindungen (1-1-7) bzw. (1-1-8) zu jeweils ca 15 %.

## Patentansprüche

1. Wirkstoffkombinationen enthaltend eine synergistisch wirksame Kombination aus mindestens einer Verbindung der allgemeinen Formel (1-1),

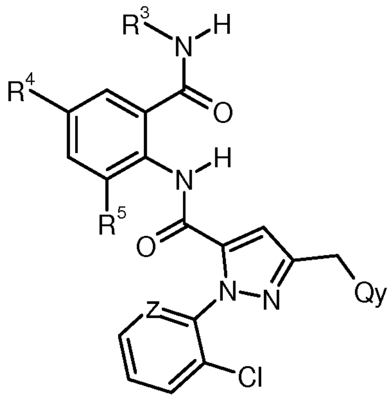

(I-1)

in welcher

$R^3$ für einen der folgenden Reste steht

H

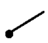 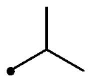 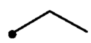 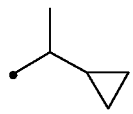

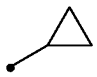 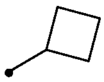 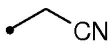 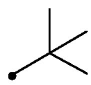 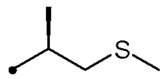

$R^4$ für Chlor und Cyano steht,
$R^4$ ebenfalls für Brom, Fluor, Jod oder Methyl steht,
$R^5$ für Methyl steht,
Z für N oder CH steht,
Qy für einen gegebenenfalls einfach oder mehrfach gleich oder verschieden substituierten heteroaromatischen Ring der Reihe Q-58 und Q-59 steht,

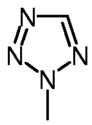
Q-58

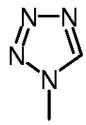
Q-59 ,

wobei die Substituenten unabhängig voneinander ausgewählt sein können aus Methyl, Ethyl, cyclo-Propyl, tert-Butyl, Difluormethyl, Trifluormethyl, Pentafluorethyl., n-Heptafluorpropyl und iso-Heptafluorpropyl,
wobei die Verbindungen der Formel (1-1) in Form von Salzen vorliegen können,
und wobei die Wirkstoffkombinationen eine Mischung von Verbindungen der allgemeinen Formel (1-1) enthalten, in welchen Qy für Q58 und Q59 steht,
und ein oder mehrere weitere Insektizide und / oder Akarizide ausgewählt aus der Gruppe (II) bestehend aus

| |
|---|
| Acrinathrin |
| Alpha-Cypermethrin |
| Betacyfluthrin |

(fortgesetzt)

| |
|---|
| Cyhalothrin |
| Cypermethrin |
| Deltamethrin |
| Esfenvalerat |
| Etofenprox |
| Fenpropathrin |
| Fenvalerat |
| Flucythrinat |
| Lambda-Cyhalothrin |
| Gamma-Cyhalothrin |
| Permethrin |
| Tau-fluvalinat |
| Transfluthrin |
| Zeta-Cypermethrin |
| Cyfluthrin |
| Bifenthrin |
| Tefluthrin |
| Eflusilanat |
| Fubfenprox |
| Pyrethrin |
| Resmethrin |
| Imidacloprid |
| Acetamiprid |
| Thiamethoxam |
| Nitenpyram |
| Thiacloprid |
| Dinotefuran |
| Clothianidin |
| Imidaclothiz |
| Chlorfluazuron |
| Diflubenzuron |
| Lufenuron |
| Teflubenzuron |
| Triflumuron |
| Novaluron |
| Flufenoxuron |
| Hexaflumuron |
| Bistrifluoron |
| Noviflumuron |

(fortgesetzt)

| |
|---|
| Buprofezin |
| Cyromazine |
| Methoxyfenozide |
| Tebufenozide |
| Halofenozide |
| Chromafenozide |
| Endosulfan |
| Fipronil |
| Ethiprole |
| Pyrafluprole |
| Pyriprole |
| Flubendiamide |
| Chlorantraniliprole (Rynaxypyr) |
| Cyazypyr |
| Emamectin |
| Emamectin benzoate |
| Abamectin |
| Ivermectin |
| Milbemectin |
| Lepimectin |
| Tebufenpyrad |
| Fenpyroximat |
| Pyridaben |
| Fenazaquin |
| Pyrimidifen |
| Tolfenpyrad |
| Dicofol |
| Cyenopyrafen |
| Cyflumetofen |
| Acequinocyl |
| Fluacrypyrin |
| Bifenazate |
| Diafenthiuron |
| Etoxazole |
| Clofentezine |
| Spinosad |
| Triarathen |
| Tetradifon |
| Propargit |

(fortgesetzt)

| |
|---|
| Hexythiazox |
| Bromopropylat |
| Chinomethionat |
| Amitraz |
| Pyrifluquinazone |
| Pymetrozine |
| Flonicamid |
| Pyriproxyfen |
| Diofenolan |
| Chlorfenapyr |
| Metaflumizone |
| Indoxacarb |
| Chlorpyrifos |
| Spirodiclofen |
| Spiromesifen |
| Spirotetramat |
| Pyridalyl |
| Spinetoram |
| Acephate |
| Triazophos |
| Profenofos |
| Fenamiphos |
| 4-{[(6-Chlorpyrid-3-yl)methyl](2,2-difluorethyl)amin o}furan-2(5H)-on |
| Cadusaphos |
| Carbaryl |
| Carbofuran |
| Ethoprophos |
| Thiodicarb |
| Aldicarb |
| Metamidophos |
| Methiocarb |
| Sulfoxaflor |
| Bacillus firmus I-1582 |
| Dichloropropen |
| Dimethoate |
| Metaldehyd |
| Methomyl |
| Cartap |
| Öl (beispielsweise Petroleum) |

(fortgesetzt)

| |
|---|
| Chloropicrin |
| Carbosulfan |
| Dichlorvos |
| Metam-Natrium |
| Phoxim |
| Monocrotophos |
| Oxamyl |
| Methidathion |
| Fenitrothion |
| Terbufos |
| Fluensulfone |
| Imicyafos |
| 11-(4-Chlor-2,6-dimethylphenyl)-12-hydroxy-1,4-dioxa-9-azadispiro[4.2.4.2 ] tetradec-11-en-10-on |
| 2-{6-[2-(5-Fluorpyridin-3-yl)-1,3-thiazol-5-yl]pyridin-2-yl}pyrimidin |

**2.** Wirkstoffkombinationen gemäß Anspruch 1, **dadurch gekennzeichnet, dass** die Insektizide und/oder Akarizide ausgewählt sind aus der Gruppe bestehend aus

| |
|---|
| Acrinathrin |
| Alpha-Cypermethrin |
| Betacyfluthrin |
| Cyhalothrin |
| Cypermethrin |
| Deltamethrin |
| Lambda-Cyhalothrin |
| Gamma-Cyhalothrin |
| Transfluthrin |
| Cyfluthrin |
| Bifenthrin |
| Tefluthrin |
| Imidacloprid |
| Acetamiprid |
| Thiamethoxam |
| Thiacloprid |
| Dinotefuran |
| Clothianidin |
| Lufenuron |
| Triflumuron |
| Novaluron |

(fortgesetzt)

| |
|---|
| Flufenoxuron |
| Buprofezin |
| Methoxyfenozide |
| Tebufenozide |
| Fipronil |
| Ethiprole |
| Flubendiamide |
| Chlorantraniliprole (Rynaxypyr) |
| Cyazypyr |
| Emamectin |
| Emamectin benzoate |
| Abamectin |
| Milbemectin |
| Tebufenpyrad |
| Fenpyroximat |
| Diafenthiuron |
| Spinosad |
| Flonicamid |
| Chlorfenapyr |
| Metaflumizone |
| Indoxacarb |
| Chlorpyrifos |
| Spirodiclofen |
| Spiromesifen |
| Spirotetramat |
| Pyridalyl |
| Spinetoram |
| Acephate |
| Triazophos |
| Profenofos |
| Fenamiphos |
| 4-{[(6-Chlorpyrid-3-yl)methyl](2,2-difluorethyl)amino}furan-2(5H)-on |
| Cadusaphos |
| Carbaryl |
| Carbofuran |
| Ethoprophos |
| Thiodicarb |
| Aldicarb |
| Metamidophos |

(fortgesetzt)

| |
|---|
| Methiocarb |
| Sulfoxaflor |
| Bacillus firmus 1-1582 |
| Dichloropropen |
| Dimethoate |
| Methomyl |
| Imicyafos |
| Fluensulfone |
| 11-(4-Chlor-2,6-dimethylphenyl)-12-hydroxy-1,4-dioxa-9-azadispiro[4.2.4.2]tetradec-11-en-10-on |
| 2-{6-[2-(5-Fluorpyridin-3-yl)-1,3-thiazol-5-yl]pyridin-2-yl}pyrimidin |

3. Wirkstoffkombinationen gemäß einem der Ansprüche 1 bis 2, wobei das Verhältnis einer Verbindung der Formel (I-1), in welchen Qy für Q58 steht, zu einer Verbindung der Formel (I-1), in welchen Qy für Q59 steht, 80:20 bis 99:1 beträgt.

4. Wirkstoffkombinationen gemäß einem der Ansprüche 1 bis 3, **dadurch gekennzeichnet, dass** das Verhältnis einer Verbindung der Formel (1-1) oder einer Mischung von Verbindungen der Formel (1-1) zu einer Verbindung der Gruppe (II) 625:1 bis 1:625 beträgt.

5. Wirkstoffkombinationen gemäß einem der Ansprüche 1 bis 4, wobei die Verbindungen der Formel (1-1) ausgewählt sind aus der Gruppe bestehend aus den Verbindungen (1-1-1) bis (1-1-60).

6. Wirkstoffkombinationen gemäß einem der Ansprüche 1 bis 4, wobei die Mischungen von Verbindungen der Formel (1-1) ausgewählt sind aus der Gruppe bestehend aus

I-1-1-/1-1-7,
1-1-2/1-1-8,
1-1-3/1-1-9,
1-1-4/1-1-10,
1-1-5/1-1-11,
1-1-6/1-1-12,
1-1-13/1-1-1-19,
1-1-14/1-1-20,
I-1-15/I-1-21,
I-1-16/I-1-22,
I-1-17/I-1-23,
I-1-18/I-1-24,
1-1-25/1-1-31,
1-1-26/1-1-32,
I-1-27/I-1-33,
1-1-28/1-1-34,
I-1-29/I-1-35,
I-1-30/I-1-36,
1-1-37/1-1-43,
1-1-38/1-1-44,
I-1-39/I-1-45,
I-1-40/I-1-46,
I-1-41/I-1-47,
I-1-42/I-1-48,
I-1-49/I-1-55,
I-1-50/I-1-56,

I-1-51/I-1-57,
I-1-52/I-1-58,
I-1-53/I-1-59,
I-1-54/I-1-60.

7. Wirkstoffkombinationen gemäß einem der Ansprüche 1 bis 6, wobei die Mischung von Verbindungen der Formel (I-1) die Mischung I-1-1/1-1-7 ist.

8. Wirkstoffkombinationen gemäß einem der Ansprüche 1 bis 6, wobei die Mischung von Verbindungen der Formel (1-1) die Mischung 1-1-2/1-1-8 ist.

9. Agrochemische Zusammensetzung enthaltend Wirkstoffkombinationen gemäß einem der Ansprüche 1 bis 8, sowie Streckmittel und/oder oberflächenaktive Stoffe.

10. Verwendung von Wirkstoffkombinationen gemäß einem der Ansprüche 1 bis 8 oder einer Zusammensetzung gemäß Anspruch 9 zur Bekämpfung tierischer Schädlinge.

11. Verfahren zur Bekämpfung tierischer Schädlinge, **dadurch gekennzeichnet, dass** man Wirkstoffkombinationen wie in Anspruch 1 bis 8 definiert oder eine Zusammensetzung gemäß Anspruch 9, auf tierische Schädlinge und/oder deren Lebensraum einwirken lässt.

12. Verfahren zur Herstellung agrochemischer Zusammensetzungen, **dadurch gekennzeichnet, dass** man Wirkstoffkombinationen wie in Anspruch 1 bis 8 definiert, mit Streckmitteln und/oder oberflächenaktiven Stoffen vermischt.

**Claims**

1. Active ingredient combinations comprising a synergistically active combination of at least one compound of the general formula (I-1)

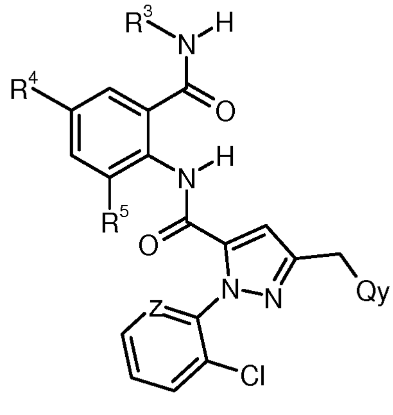

(I-1)

in which

$R^3$ is one of the following radicals

H

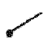 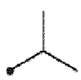 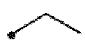 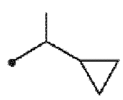

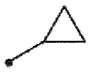 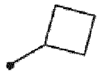 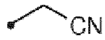 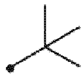 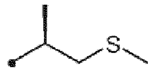

$R^4$ is chlorine and cyano,
$R^4$ is likewise bromine, fluorine, iodine or methyl,
$R^5$ is methyl,
Z is N or CH,
Qy is a heteroaromatic ring, optionally mono- or polysubstituted identically or differently, from the group of Q-58 and Q-59, where the substituents may each independently be selected from

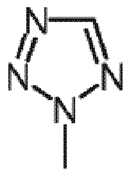
Q-58

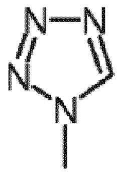
Q-59

where the compounds of the formula (I-1) may be present in the form of salts, methyl, ethyl, cyclo-propyl, tert-butyl, difluoromethyl, trifluoromethyl, pentafluoroethyl, n-heptafluoropropyl and isoheptafluoropropyl and where the active ingredient combinations comprise a mixture of compounds of the general formula (I-1) in which Qy is Q58 and Q59, and one or more further insecticides and/or acaricides selected from group (II) consisting of

| |
|---|
| acrinathrin |
| alpha-cypermethrin |
| betacyfluthrin |
| cyhalothrin |
| cypermethrin |
| deltamethrin |
| esfenvalerat |
| etofenprox |
| fenpropathrin |
| fenvalerate |
| flucythrinate |
| lambda-cyhalothrin |
| gamma-cyhalothrin |
| permethrin |
| tau-fluvalinate |
| transfluthrin |
| zeta-cypermethrin |
| cyfluthrin |
| bifenthrin |
| tefluthrin |
| eflusilanate |

(continued)

| |
|---|
| fubfenprox |
| pyrethrin |
| resmethrin |
| imidacloprid |
| acetamiprid |
| thiamethoxam |
| nitenpyram |
| thiacloprid |
| dinotefuran |
| clothianidin |
| imidaclothiz |
| chlorfluazuron |
| diflubenzuron |
| lufenuron |
| teflubenzuron |
| triflumuron |
| novaluron |
| flufenoxuron |
| hexaflumuron |
| bistrifluoron |
| noviflumuron |
| buprofezin |
| cyromazine |
| methoxyfenozide |
| tebufenozide |
| halofenozide |
| chromafenozide |
| endosulfan |
| fipronil |
| ethiprole |
| pyrafluprole |
| pyriprole |
| flubendiamide |
| chlorantraniliprole (Rynaxypyr) |
| cyazypyr |
| emamectin |
| emamectin benzoate |
| abamectin |
| ivermectin |

(continued)

| |
|---|
| milbemectin |
| lepimectin |
| tebufenpyrad |
| fenpyroximate |
| pyridaben |
| fenazaquin |
| pyrimidifen |
| tolfenpyrad |
| dicofol |
| cyenopyrafen |
| cyflumetofen |
| acequinocyl |
| fluacrypyrin |
| bifenazate |
| diafenthiuron |
| etoxazole |
| clofentezine |
| spinosad |
| triarathen |
| tetradifon |
| propargite |
| hexythiazox |
| bromopropylate |
| chinomethionat |
| amitraz |
| pyrifluquinazone |
| pymetrozine |
| flonicamid |
| pyriproxyfen |
| diofenolan |
| chlorfenapyr |
| metaflumizone |
| indoxacarb |
| chlorpyrifos |
| spirodiclofen |
| spiromesifen |
| spirotetramat |
| pyridalyl |
| spinetoram |

(continued)

| |
|---|
| acephate |
| triazophos |
| profenofos |
| fenamiphos |
| 4-{[(6-chloropyrid-3-yl)methyl] (2,2-difluoroethyl)amin o}furan-2(5H)-one |
| cadusaphos |
| carbaryl |
| carbofuran |
| ethoprophos |
| thiodicarb |
| aldicarb |
| metamidophos |
| methiocarb |
| sulfoxaflor |
| Bacillus firmus I-1582 |
| dichloropropene |
| dimethoate |
| metaldehyde |
| methomyl |
| cartap |
| oil (for example petroleum) |
| chloropicrin |
| carbosulfan |
| dichlorvos |
| metam-sodium |
| phoxim |
| monocrotophos |
| oxamyl |
| methidathion |
| fenitrothion |
| terbufos |
| fluensulfone |
| imicyafos |
| 11-(4-chloro-2,6-dimethylphenyl)-12-hydroxy-1,4-dioxa-9-azadispiro[4.2.4.2] tetradec-11-en-10-one |
| 2-{6-[2-(5-fluoropyridin-3-yl)-1,3-thiazol-5-yl]pyridin-2-yl}pyrimidine |

**2.** Active ingredient combinations according to Claim 1, **characterized in that** the insecticides and/or acaricides are selected from the group consisting of

| |
|---|
| acrinathrin |
| alpha-cypermethrin |
| betacyfluthrin |
| cyhalothrin |
| cypermethrin |
| deltamethrin |
| lambda-cyhalothrin |
| gamma-cyhalothrin |
| transfluthrin |
| cyfluthrin |
| bifenthrin |
| tefluthrin |
| imidacloprid |
| acetamiprid |
| thiamethoxam |
| thiacloprid |
| dinotefuran |
| clothianidin |
| lufenuron |
| triflumuron |
| novaluron |
| flufenoxuron |
| buprofezin |
| methoxyfenozide |
| tebufenozide |
| fipronil |
| ethiprole |
| flubendiamide |
| chlorantraniliprole (Rynaxypyr) |
| cyazypyr |
| emamectin |
| emamectin benzoate |
| abamectin |
| milbemectin |
| tebufenpyrad |
| fenpyroximate |
| diafenthiuron |
| spinosad |

(continued)

| |
|---|
| flonicamid |
| chlorfenapyr |
| metaflumizone |
| indoxacarb |
| chlorpyrifos |
| spirodiclofen |
| spiromesifen |
| spirotetramat |
| pyridalyl |
| spinetoram |
| acephate |
| triazophos |
| profenofos |
| fenamiphos |
| 4-{[(6-chloropyrid-3-yl)methyl] (2,2-difluoroethyl)amin o}furan-2(5H)-one |
| cadusaphos |
| carbaryl |
| carbofuran |
| ethoprophos |
| thiodicarb |
| aldicarb |
| metamidophos |
| methiocarb |
| sulfoxaflor |
| Bacillus firmus I-1582 |
| dichloropropene |
| dimethoate |
| methomyl |
| imicyafos |
| fluensulfone |
| 11-(4-chloro-2,6-dimethylphenyl)-12-hydroxy-1,4-dioxa-9-azadispiro[4.2.4.2] |
| tetradec-11-en-10-one |
| 2-{6-[2-(5-fluoropyridin-3-yl)-1,3-thiazol-5-yl]pyridin-2-yl}pyrimidine |

**3.** Active ingredient combinations according to either of Claims 1 and 2, where the ratio of a compound of the formula (I-1) in which Qy is Q58 to a compound of the formula (I-1) in which Qy is Q59 is 80:20 to 99:1.

**4.** Active ingredient combinations according to any of Claims 1 to 3, **characterized in that** the ratio of a compound of the formula (I-1) or of a mixture of compounds of the formula (I-1) to a compound of group (II) is 625:1 to 1:625.

**5.** Active ingredient combinations according to any of Claims 1 to 4, wherein the compounds of the formula (I-1) are

selected from the group consisting of the compounds (I-1-1) to (I-1-60).

**6.** Active ingredient combinations according to any of Claims 1 to 4, wherein the mixtures of compounds of the formula (I-1) are selected from the group consisting of

I-1-1-/1-1-7,
1-1-2/1-1-8,
1-1-3/1-1-9,
1-1-4/1-1-10,
1-1-5/1-1-11,
1-1-6/1-1-12,
1-1-13/1-1-1-19,
1-1-14/1-1-20,
I-1-15/I-1-21,
I-1-16/I-1-22,
I-1-17/I-1-23,
I-1-18/I-1-24,
1-1-25/1-1-31,
1-1-26/1-1-32,
I-1-27/I-1-33,
1-1-28/1-1-34,
I-1-29/I-1-35,
I-1-30/I-1-36,
1-1-37/1-1-43,
1-1-38/1-1-44,
I-1-39/I-1-45,
I-1-40/I-1-46,
I-1-41/I-1-47,
I-1-42/I-1-48,
I-1-49/I-1-55,
I-1-50/I-1-56,
I-1-51/I-1-57,
I-1-52/I-1-58,
I-1-53/I-1-59,
I-1-54/I-1-60.

**7.** Active ingredient combinations according to any of Claims 1 to 6, wherein the mixture of compounds of the formula (I-1) is the mixture I-1-1/1-1-7.

**8.** Active ingredient combinations according to any of Claims 1 to 6, wherein the mixture of compounds of the formula (I-1) is the mixture I-1-2/1-1-8.

**9.** Agrochemical composition comprising active ingredient combinations according to any of Claims 1 to 8, and extenders and/or surfactants.

**10.** Use of active ingredient combinations according to any of Claims 1 to 8 or of a composition according to Claim 9 for control of animal pests.

**11.** Method for controlling animal pests, **characterized in that** active ingredient combinations as defined in Claims 1 to 8 or a composition according to Claim 9 is allowed to act on animal pests and/or their habitat.

**12.** Process for producing agrochemical compositions, **characterized in that** active ingredient combinations as defined in Claims 1 to 8 are mixed with extenders and/or surfactants.

**Revendications**

**1.** Associations de substances actives contenant une association synergiquement efficace d'au moins un composé

de formule générale (1-1),

(I),

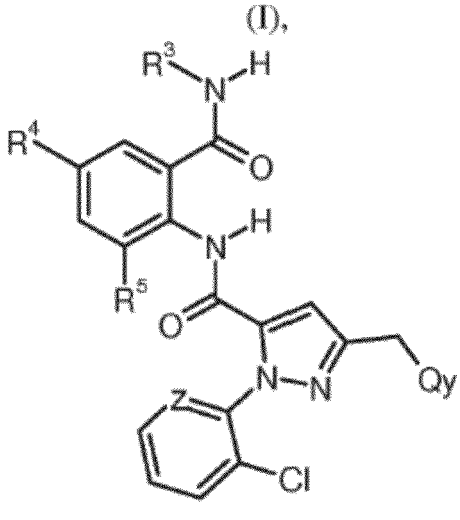

(I-1)

dans laquelle

R³ représente l'un des radicaux suivants

H

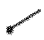 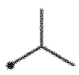 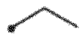 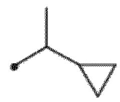

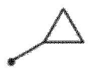 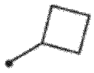 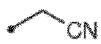 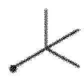 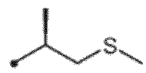

$R^4$ représente un atome de chlore ou le groupe cyano,
$R^4$ représente également un atome de brome, fluor, iode ou le groupe méthyle,
$R^5$ représente le groupe méthyle,
Z représente N ou CH,
Qy représente un cycle hétéroaromatique de la série Q-58 et Q-59, portant éventuellement un ou plusieurs substituants identiques ou différents,

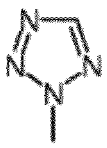 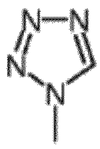

Q-58 Q-59

les substituants pouvant être choisis indépendamment les uns des autres parmi méthyle, éthyle, cyclopropyle, tert-butyle, difluoro-méthyle, trifluorométhyle, pentafluoroéthyle, n-heptafluoropropyle et iso-heptafluoropropyle,
les composés de formule (I-1) pouvant se trouver sous forme de sels,
et les associations de substances actives contenant un mélange de composés de formule générale (1-1), dans laquelle Qy représente Q58 et Q59,
et un ou plusieurs autres insecticides et/ou acaricides choisis dans le groupe (II) consistant en

| |
|---|
| acrinathrine |
| alpha-cyperméthrine cyperméthrine |
| bêta-cyfluthrine |
| cyhalothrine |
| cyperméthrine |
| deltaméthrine |
| esfenvalérate |
| étofenprox |
| fenpropathrine |
| fenvalérate |
| flucythrinate |
| lambda-cyhalothrine |
| gamma-cyhalothrine |
| perméthrine |
| tau-fluvalinate |
| transfluthrine |
| zêta-cyperméthrine |
| cyfluthrine |
| bifenthrine |
| téfluthrine |
| éflusilanate |
| fubfenprox |
| pyréthrine |
| resméthrine |
| imidaclopride |
| acétamipride |
| thiaméthoxame |
| nitenpyram |
| thiaclopride |
| dinotéfuran |
| clothianidine |
| imidaclothiz |
| chlorfluazuron |
| diflubenzuron |
| leufénuron |
| téflubenzuron |
| triflumuron |
| novaluron |

(suite)

| |
|---|
| flufénoxuron |
| hexaflumuron |
| bistrifluron |
| noviflumuron |
| buprofézine |
| cyromazine |
| méthoxyfénozide |
| tébufénozide |
| halofénozide |
| chromfénozide |
| endosulfan |
| fipronil |
| éthiprol |
| pyrafluprole |
| pyriprole |
| flubendiamide |
| chlorantraniliprole (rynaxypyr) |
| cyazypyr |
| émamectine |
| émamectine benzoate |
| abamectine |
| ivermectine |
| milbémectine |
| lépimectine |
| tébufenpyrad |
| fenpyroximate |
| pyridabène |
| fénazaquin |
| pyrimidifène |
| tolfenpyrad |
| dicofol |
| cyénopyrafène |
| cyflumétofène |
| acéquinocyl |
| fluacrypyrime |
| bifénazate |
| diafenthiuron |
| éthoxazole |
| chlofentézine |

(suite)

| |
|---|
| spinosad |
| triarathène |
| tétradifon |
| propargite |
| hexythiazox |
| bromopropylate |
| chinométhionate |
| amitraz |
| pyrifluquinazone |
| pymétrozine |
| flonicamide |
| pyriproxyfène |
| diofénolan |
| chlorfénapyr |
| métaflumizone |
| indoxacarbe |
| chlorpyrifos |
| spirodiclofène |
| spiromésifène |
| spirotétramate |
| pyridalyl |
| spinétorame |
| acéphate |
| triazophos |
| profénofos |
| fénamiphos |
| 4-{[(6-chloropyrid-3-yl)méthyl](2,2-difluoréthyl)amino}furan-2(5H)-one |
| cadusafos |
| carbaryl |
| carbofuran |
| éthoprophos |
| thiodicarbe |
| aldicarbe |
| méthamidophos |
| méthiocarbe |
| sulfoxaflor |
| Bacillus firmus 1-1582 |
| dichloropropène |
| diméthoate |

(suite)

| |
|---|
| métaldéhyde |
| méthomyl |
| cartap |
| huile (par exemple pétrole) |
| chloropicrine |
| carbosulfan |
| dichlorvos |
| métam-sodium |
| phoxim |
| monocrotophos |
| oxamyl |
| méthidathion |
| fénitrothion |
| terbufos |
| fluensulfone |
| imicyafos |
| 11-(4-chloro-2,6-diméthylphényl)-12-hydroxy-1,4-dioxa-9-azadispiro-[4.2.4.2]-tétradéc-11-én-10-one |
| 2-{6-[2-(5-fluoropyridin-3-yl)-1,3-thiazol-5-yl]pyridin-2-yl}pyrimidine |

**2.** Associations de substances actives selon la revendication 1, **caractérisées en ce que** les insecticides et/ou acaricides sont choisis dans le groupe consistant en

| |
|---|
| acrinathrine |
| alpha-cyperméthrine cyperméthrine |
| bêta-cyfluthrine |
| cyhalothrine |
| cyperméthrine |
| deltaméthrine |
| lambda-cyhalothrine |
| gamma-cyhalothrine |
| transfluthrine |
| cyfluthrine |
| bifenthrine |
| téfluthrine |
| imidaclopride |
| acétamipride |
| thiaméthoxame |
| thiaclopride |
| dinotéfuran |

(suite)

| |
|---|
| clothianidine |
| leufénuron |
| triflumuron |
| novaluron |
| flufénoxuron |
| buprofézine |
| méthoxyfénozide |
| tébufénozide |
| fipronil |
| éthiprol |
| flubendiamide |
| chlorantraniliprole (rynaxypyr) |
| cyazypyr |
| émamectine |
| émamectine benzoate |
| abamectine |
| milbémectine |
| tébufenpyrad |
| fenpyroximate |
| diafenthiuron |
| spinosad |
| flonicamide |
| chlorfénapyr |
| métaflumizone |
| indoxacarbe |
| chlorpyrifos |
| spirodiclofène |
| spiromésifène |
| spirotétramate |
| pyridalyl |
| spinétorame |
| acéphate |
| triazophos |
| profénofos |
| fénamiphos |
| 4-{[(6-chloropyrid-3-yl)méthyl](2,2-difluoréthyl)amino}furan-2(5H)-one |
| cadusafos |
| carbaryl |
| carbofuran |

(suite)

| |
|---|
| éthoprophos |
| thiodicarbe |
| aldicarbe |
| méthamidophos |
| méthiocarbe |
| sulfoxaflor |
| Bacillus firmus 1-1582 |
| dichloropropène |
| diméthoate |
| méthomyl |
| imicyafos |
| fluensulfone |
| 11-(4-chloro-2,6-diméthylphényl)-12-hydroxy-1,4-dioxa-9-azadispiro-[4.2.4.2]-tétradéc-11-én-10-one |
| 2-{6-[2-(5-fluoropyridin-3-yl)-1,3-thiazol-5-yl]pyridin-2-yl}pyrimidine |

**3.** Associations de substances actives selon l'une quelconque des revendications 1 et 2, dans lesquelles le rapport d'un composé de formule (1-1), dans laquelle Qy représente Q58, à un composé de formule (1-1), dans laquelle Qy représente Q59, vaut de 80:20 à 99:1.

**4.** Associations de substances actives selon l'une quelconque des revendications 1 à 3, **caractérisées en ce que** le rapport d'un composé de formule (I-1) ou d'un mélange de composés de formule (I-1) à un composé de formule (II) vaut de 625:1 à 1:625.

**5.** Associations de substances actives selon l'une quelconque des revendications 1 à 4, dans lesquelles les composés de formule (I-1) sont choisis dans le groupe constitué par les composés (1-1-1) à (1-1-60).

**6.** Associations de substances actives selon l'une quelconque des revendications 1 à 4, dans lesquelles les mélanges de composés de formule (I-1) sont choisis dans le groupe constitué par

I-1-1-/1-1-7,
1-1-2/1-1-8,
1-1-3/1-1-9,
1-1-4/1-1-10,
1-1-5/1-1-11,
1-1-6/1-1-12,
1-1-13/1-1-1-19,
1-1-14/1-1-20,
I-1-15/I-1-21,
1-1-16/1-1-22,
I-1-17/I-1-23,
I-1-18/I-1-24,
1-1-25/1-1-31,
1-1-26/1-1-32,
I-1-27/ 1-1-33,
1-1-28/1-1-34,
1-1-29/1-1-35,
1-1-30/1-1-36,
1-1-37/1-1-43,
1-1-38/1-1-44,
I-1-39/I-1-45,

I-1-40/I-1-46,
1-1-41/1-1-47,
I-1-42/I-1-48,
1-1-49/1-1-55,
1-1-50/1-1-56,
1-1-51/1-1-57,
I-1-52/I-1-58,
I-1-53/I-1-59,
I-1-54/1-1-60.

**7.** Associations de substances actives selon l'une quelconque des revendications 1 à 6, dans lesquelles le mélange de composés de formule (I-1) est le mélange I-1-1/I-1-7.

**8.** Associations de substances actives selon l'une quelconque des revendications 1 à 6, dans lesquelles le mélange de composés de formule (I-1) est le mélange I-1-2/I-1-8.

**9.** Composition agrochimique contenant des associations de substances actives selon l'une quelconque des revendications 1 à 8, ainsi que des diluants et/ou des substances tensioactives.

**10.** Utilisation d'associations de substances actives selon l'une quelconque des revendications 1 à 8 ou d'une composition selon la revendication 9, pour la lutte contre des ravageurs animaux.

**11.** Procédé pour la lutte contre des ravageurs animaux, **caractérisé en ce qu'**on fait agir sur des ravageurs animaux et/ou leur habitat des associations de substances actives telles que définies dans l'une quelconque des revendications 1 à 8 ou une composition selon la revendication 9.

**12.** Procédé pour la préparation de compositions agrochimiques, **caractérisé en ce qu'**on mélange des associations de substances actives telles que définies dans l'une quelconque des revendications 1 à 8 avec des diluants et/ou des substances tensioactives.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2007144100 A


### In der Beschreibung aufgeführte Nicht-Patentliteratur


- The Pesticide Manual. British Crop Protection Council. 2006
- **S.R. COLBY.** *Weeds,* 1967, vol. 15, 20-22